(19) Europäisches Patentamt
European Patent Office
Office européen des brevets

(11) Publication number : **0 426 804 B1**

(12) # EUROPEAN PATENT SPECIFICATION

(45) Date of publication of patent specification : **19.01.94 Bulletin 94/03**

(51) Int. Cl.[5] : **C07C 217/30,** A61K 31/135, A61K 31/19, A61K 31/215, C07D 263/24, C07C 217/22

(21) Application number : **90907243.1**

(22) Date of filing : **30.04.90**

(86) International application number : **PCT/GB90/00668**

(87) International publication number : **WO 90/13535 15.11.90 Gazette 90/26**

(54) **2-(2-HYDROXY-3-PHENOXYPROPYLAMINO)-ETHOXYBENZENE DERIVATIVES.**

The file contains technical information submitted after the application was filed and not included in this specification

(30) Priority : **05.05.89 GB 8910374**

(43) Date of publication of application : **15.05.91 Bulletin 91/20**

(45) Publication of the grant of the patent : **19.01.94 Bulletin 94/03**

(84) Designated Contracting States : **AT BE CH DE DK ES FR GB IT LI LU NL SE**

(56) References cited :
**EP-A- 0 070 133**
**EP-A- 0 210 849**
**GB-A- 1 245 148**
**US-A- 4 263 323**

(73) Proprietor : **IMPERIAL CHEMICAL INDUSTRIES PLC**
**Imperial Chemical House, Millbank**
**London SW1P 3JF (GB)**

(72) Inventor : **HOWE, Ralph**
**Little Beck Upton Macclesfield**
**Cheshire SK10 3HL (GB)**
Inventor : **LE COUNT, David, James**
**1 Vernon Avenue Congleton**
**Cheshire CW12 3AZ (GB)**
Inventor : **RAO, Balbir, Singh**
**3 Elmore Close Holmes Chapel Nr Crewe**
**Cheshire CW4 7HW (GB)**

(74) Representative : **Denerley, Paul Millington, Dr. et al**
**ICI Group Patents Services Dept. PO Box 6**
**Shire Park Bessemer Road**
**Welwyn Garden City Herts, AL7 1HD (GB)**

**EP 0 426 804 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 2-hydroxy-3-phenoxypropylamino compounds and in particular to such compounds containing a phenylacetyl group and certain derivatives thereof. This invention further relates to processes and intermediates for their preparation, to their use in methods of therapy and to pharmaceutical compositions containing them. Administration of the compounds of this invention to warm-blooded animals provides a thermogenic effect, that is thermogenesis is stimulated and administration of the compounds is of use, for example, in the treatment of obesity and related conditions such as obesity associated with mature onset diabetes.

UK Patent 1245148 claims compounds of the general formula:

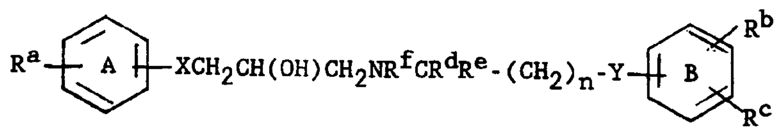

wherein $R^a$ is hydrogen, halogen, or a lower alkyl, phenyl, lower alkoxy or phenyl substituted lower alkyl group;
$R^b$ is hydrogen or up to two halogen, lower alkyl or lower alkoxy substituents;
$R^c$ is an electron-withdrawing polar substituent which may include groups such as carboxy, lower alkoxycarbonyl, formyl, lower alkanoyl, carbamoyl, sulpho, sulphino, alkoxysulphonyl, alkoxysulphinyl, sulphamoyl, cyano, azido, nitro or trifluoromethyl or any such group separated from the phenyl ring by a methylene or ethylene group;
$R^d$ and $R^e$ each represent hydrogen or a lower alkyl group;
$R^f$ represents hydrogen or a lower alkyl, alkanoyl or benzyl group;
either of the benzene rings A and B may be optionally substituted further by a -CH=CH-CH=CH- radical to form a naphthyl group;
X is oxygen or sulphur;
and either Y is oxygen, sulphur or sulphinyl, sulphonyl or imino group and n is 1, 2 or 3; or Y is a direct bond and n is 0,1,2,3 or 4;
wherein the term "lower" menas up to 4 carbon atoms.

These compounds are described as having the ability to block the beta-adrenergic receptors and are stated to be useful in the curative or prophylactic treatment of cardiac disorders such as angina pectoris and cardiac arrhythmias and in the treatment of hypertension. Biological data are reported describing the cardiac effects of various compounds within the claims.

We have now discovered a small class of compounds related to those of the above general formula which compounds, surprisingly, provide significant thermogenic effects at doses which cause relatively few side-effects. It is understood that selectivity of thermogenic effect, for example lack of cardiac side-effects, is an important requirement for a useful therapeutic agent in the treatment of, for example, obesity and related conditions.

Compounds of the formula:

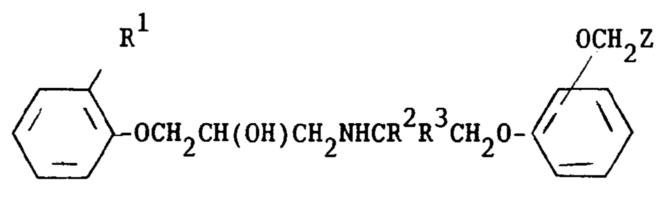

in which $R^1$ is hydrogen or fluoro, $R^2$ and $R^3$ are hydrogen or $C_{1-3}$alkyl, Z is $-CH_2OH$ or a group $-COR^4$ wherein $R^4$ is hydroxy, $NH_2$ or $C_{1-6}$alkoxy are known as thermogenic agents from EP-A-210849.

Accordingly the present invention provides a compound of the formula (I):

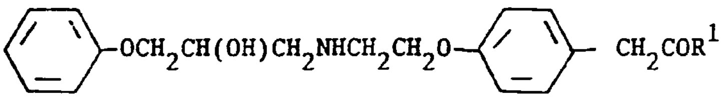

(I)

wherein $R^1$ is hydroxy or $C_{1-10}$alkyl, or an in vivo hydrolysable ester or a pharmaceutically acceptable salt thereof.

In one aspect $R^1$ is hydroxy. In another aspect $R^1$ is $C_{1-10}$ for example methyl, ethyl, n-propyl, isopropyl, n-butyl, n-pentyl n-hexyl and n-heptyl. Preferably $R^1$ is $C_{1-6}$alkyl for example, methyl ethyl, n-propyl, isopropyl, n-butyl and n-hexyl.

The compounds of the formula (I) are basic and may be isolated and used either in the form of the free base or of a pharmaceutically acceptable acid-addition salt thereof. In addition, compounds wherein $R^1$ is hydroxy are amphoteric and may be used in the zwitterionic form, or as a pharmaceutically acceptable acid addition salt, or as a salt with a base affording a pharmaceutically acceptable cation. Particular examples of pharmaceutically acceptable acid-addition salts include, for example, salts with inorganic acids such as hydrohalides (especially hydrochlorides or hydrobromides), sulphates and phosphates, and salts with organic acids such as succinates, citrates, lactates, tartrates, oxalates and salts derived from acidic water-soluble polymers. Particular examples of salts with bases affording a pharmaceutically acceptable cation include, for example, alkali metal and alkaline earth metal salts, such as sodium, potassium, calcium and magnesium salts, and ammonium salts and salts with suitable organic bases such as triethanolamine.

In vivo hydrolysable esters may be formed at the carboxy group (-COOH) and/or at the hydroxy group (-OH). In vivo hydrolysable esters are those pharmaceutically acceptable esters that hydrolyse in the human or animal body to produce the parent hydroxy compound. Such esters can be identified by administering, for example intravenously to a test animal, the compound under test and subsequently examining the test animal's body fluids. Suitable in vivo hydrolysable esters for carboxy include the $C_{1-6}$alkyl esters. Suitable in vivo hydrolysable esters for hydroxy include the $C_{1-6}$alkanoyl esters.

It will be appreciated that the compounds of the formula (I) contain one or more asymmetric carbon atoms and can exist as optically active enantiomers or as optically inactive racemates. The present invention encompasses any enantiomer, racemate and/or (when two or more asymmetric carbon atoms are present) diastereoisomer, which when administered in a therapeutic amount provides a thermogenic effect in warm-blooded animals, it being well known in the chemical art how to prepare individual enantiomers, for example by resolution of the racemate or by stereospecific synthesis, and how to determine the thermogenic properties, for example, using the standard tests described hereinafter. It is preferred that the compounds of the present invention are provided in the (S) absolute configuration at the -CH(OH)- group (under the Cahn-Prelog-Ingold rules) which generally corresponds to the laevorotatory optically active form (-) in this class of compounds.

Particular compounds of the present invention include:

4-[2-(2-hydroxy-3-phenoxypropylamino)ethoxy]phenylacetic acid,
(S)-4-[2-(2-hydroxy-3-phenoxypropylamino)ethoxy]phenylacetic acid,
1-[4-(2-(2-hydroxy-3-phenoxypropylamino)ethoxy)phenyl]propan-2-one,
(S)-1-[4-(2-(2-hydroxy-3-phenoxypropylamino)ethoxy)phenyl]-propan-2-one,
1-[4-(2-(2-hydroxy-3-phenoxypropylamino)ethoxy)phenyl]butan-2-one,
(S)-1-[4-(2-(2-hydroxy-3-phenoxypropylamino)ethoxy)phenyl]butan-2-one,
1-[4-(2-(2-hydroxy-3-phenoxypropylamino)ethoxy)phenyl]pentan-2-one,
(S)-1-[4-(2-(2-hydroxy-3-phenoxypropylamino)ethoxy)phenyl]-pentan-2-one,
1-[4-(2-(2-hydroxy-3-phenoxypropylamino)ethoxy)phenyl]-3-methylbutan-2-one,
1-[4-(2-(2-hydroxy-3-phenoxypropylamino)ethoxy)phenyl]hexan-2-one,
1-[4-(2-(2-hydroxy-3-phenoxypropylamino)ethoxy)phenyl]-4-methylpentan-2-one,
1-[4-(2-(2-hydroxy-3-phenoxypropylamino)ethoxy)phenyl]heptan-2-one,
1-[4-(2-(2-hydroxy-3-phenoxypropylamino)ethoxy)phenyl]octan-2-one and
1-[4-(2-(2-hydroxy-3-phenoxypropylamino)ethoxy)phenyl]nonan-2-one.

In order to use a compound of the present invention or an in vivo hydrolysable ester or pharmaceutically acceptable salt thereof for the therapeutic treatment of warm-blooded mammals including humans, in particular for treating obesity, it is normally formulated in accordance with standard pharmaceutical practice as a pharmaceutical composition.

Therefore in another aspect the present invention provides a pharmaceutical composition which comprises a compound of the formula (I) or an in vivo hydrolysable ester or a pharmaceutically acceptable salt thereof and a pharmaceutically acceptable carrier.

The pharmaceutical compositions of this invention may be administered in standard manner for example by oral or parenteral administration. For these purposes they may be formulated by means known to the art into the form of, for example, tablets, capsules, pills, powders, aqueous or oily solutions or suspensions, emulsions, and sterile injectable aqueous or oily solutions or suspensions.

In general compositions for oral administration are preferred.

The compositions may be obtained using standard excipients and procedures well known in the art. A unit dose form such as a tablet or capsule will usually contain, for example 0.1-250 mg of active ingredient. The compositions may also contain other active ingredients known for use in the treatment of obesity and related conditions, for example appetite suppressants, vitamins and hypoglycaemic agents.

In one aspect of the present invention, a compound of the formula (I) may be formulated for oral administration in a sustained (or delayed) release composition, for example a matrix tablet formulation comprising insoluble or swellable polymeric filler, or a coated spheroid formulation.

When used to produce thermogenic effects in warm-blooded animals including man, a compound of the formula (I) or an in vivo hydrolysable ester or pharmaceutically acceptable salt thereof as appropriate, will be administered so that a dose in the general range 0.002-20 mg/kg, and preferably in the range 0.02-10 mg/kg, is administered daily, given in a single dose or divided doses as necessary. However, it will be appreciated by those skilled in the art that dosage will necessarily be varied as appropriate, depending on the severity of the condition under treatment and on the age and sex of the patient and according to known medical principles.

In addition the compounds of the present invention lower triglyceride levels and cholesterol levels and raise high density lipoprotein levels and are therefore of use in combatting medical conditions wherein such lowering (and raising) is thought to be beneficial. Thus they may be used in the treatment of hypertriglyceridaemia, hypercholesterolaemia and conditions of low HDL (high density lipoprotein) levels in addition to the treatment of atherosclerotic disease such as of coronary, cerebrovascular and peripheral arteries, cardiovascular disease and related conditions.

Accordingly in another aspect the present invention provides a method of lowering triglyceride and/or cholesterol levels and/or increasing high density lipoprotein levels which comprises administering, to an animal in need thereof, a therapeutically effective amount of a compound of the formula (I) or in vivo hydrolysable ester or pharmaceutically acceptable salt thereof. In a further aspect the present invention provides a method of treating atherosclerosis which comprises administering, to an animal in need thereof, a therapeutically effective amount of a compound of the formula (I) or in vivo hydrolysable ester or pharmaceutically acceptable salt thereof. The compositions are formulated and administered in the same general manner as detailed above for producing a thermogenic effect. They may also contain other active ingredients known for use in the treatment of atherosclerosis and related conditions, for example fibrates such as clofibrate, bezafibrate and gemfibrozil; inhibitors of cholesterol biosynthesis such as HMG-CoA reductase inhibitors for example lovastatin, simvastatin and pravastatin;
inhibitors of cholesterol absorption for example beta-sitosterol and (acyl CoA:cholesterol acyltransferase) inhibitors for example melinamide; anion exchange resins for example cholestyramine, colestipol or a dialkylaminoalkyl derivatives of a cross-linked dextran; nicotinyl alcohol, nicotinic acid or a salt thereof; vitamin E; and thyromimetics.

In a further aspect the present invention provides a process for preparing a compound of the formula (I) or an in vivo hydrolysable ester or a pharmaceutically acceptable salt thereof which process comprises:

a) reacting a compound of the formula (II) or (III) with a compound of the formula (IV):

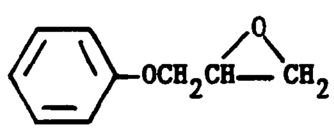

(II)

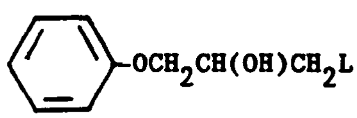

(III)

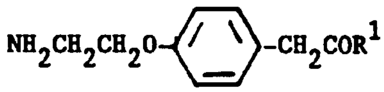

(IV)

wherein $R^1$ is as hereinbefore defined and L is a displaceable group; or

b) hydrolysis of a compound of the formula (V):

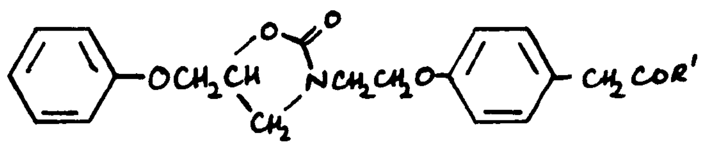

(v)

wherein $R^1$ is as hereinbefore defined; or

c) for compounds of the formula (I) wherein $R^1$ is hydroxy, hydrolysing a compound of the formula (VI):

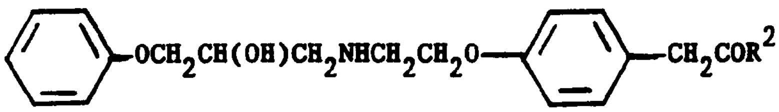

(VI)

wherein $R^2$ is a hydrolysable group;

d) reacting a compound of the formula (VII) with a compound of the formula (VIII):

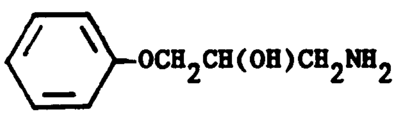

(VII)

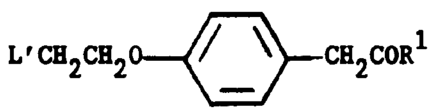

(VIII)

wherein $R^1$ is as hereinbefore defined and L' is a leaving group; or

e) deprotecting a compound of the formula (IX):

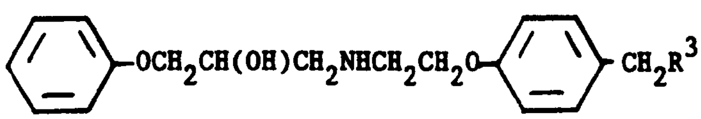

(IX)

wherein $R^3$ is a protected derivative of a group $-COR^1$:

and if necessary thereafter forming an in vivo hydrolysable ester or a pharmaceutically acceptable salt.

The reaction between a compound of the formulae (II) or (III) and a compound of the formula (IV) may be performed in a suitable solvent for example an alcohol such as ethanol or propan-2-ol, at a temperature in the range for example 10°C to 110°C and most conveniently at or near the boiling-point of the reaction mixture. In the compound of the formula (III) L may be for example halogen such as chloro or bromo or sulphonyloxy such as toluenesulphonyloxy or methanesulphonyloxy.

The compounds of the formula (IV) are prepared in any convenient manner known to those skilled in the art. For example they may be conveniently prepared by reacting compound (X) with a compound of the formula (XI):

$NH_2CH_2CH_2OH$ (X)

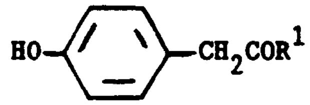

(XI)

For example this reaction may be performed using the Mitsunobu reaction with diethyl azodicarboxylate and triphenylphosphine. Desirably the amino function (and carboxy-function, if present) is protected during this reaction and subsequently deprotected in conventional manner. Examples of suitable protecting groups for the amino function include the phthaloyl and t-butoxycarbonyl groups. The compounds of the formula(XI) may be prepared according to the methods described in the Examples hereinafter.

The compound of the formula (V) may be hydrolysed to a compound of the formula (I) under conditions known in the beta-blocker art; for example via alkaline hydrolysis in a suitable solvent.

The compounds of the formula (V) may be prepared by the reaction of a compound of the formula (XI) with a compound of the formula (XII):

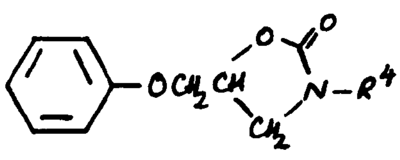

(XII)

wherein $R^4$ is a group $-CH_2CH_2OH$. This reaction may be performed in any conventional manner for example by a method analogous to the reaction of the compounds of the formulae (X) and (XI). In an alternative the compounds of the formula (V) may be prepared by the reaction of a compound of the formula (XII) wherein $R^4$ is hydrogen with a compound of the formula (VIII) as hereinbefore described. In a further alternative the compounds of the formula (V) may be prepared by the reaction of a compound of the formula (II) with a compound of the formula (XIII):

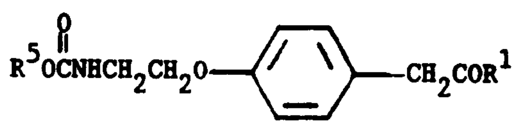

(XIII)

wherein $R^1$ is as hereinbefore defined and $R^5O$- is a leaving group, for example $R^5O$- is $C_{1-4}$alkoxy.

The compound of the formula (XII) wherein $R^4$ is $-CH_2CH_2OH$ may be prepared for example by reaction of a compound of the formula (II) with an N-alkoxycarbonyl derivative of a compound of the formula (X) wherein the hydroxy group is optionally protected, for example the tetrahydropyranyl ether of t-butoxycarbonylaminoethanol. The compounds of the formula (XII) wherein $R^4$ is hydrogen are obtainable in conventional manner. The compounds of the formulae (VIII) and (XIII) may be obtained by alkylation of the compounds of the formula (XI) in conventional manner.

The reaction between the compounds of the formulae (VII) and (VIII) is conveniently performed under conditions analogous to the reaction between compounds of the formulae (III) and (IV). L' may have similar values as recited hereinabove for L.

In the compounds of the formula (VI) examples of hydrolysable groups $R^2$ include $C_{1-6}$ alkoxy and $-NH_2$ groups so that $-COR^2$ represents a $C_{1-6}$alkyl ester or primary amide function. Such groups may be hydrolysed (acidic, basic, enzymatic) to a group $-CO_2H$ under conventional conditions. Suitable acid conditions are for example a strong mineral acid such as hydrochloric, sulphuric or phosphoric acid, conveniently at a temperature in the range, for example, 20° to 110°C and in a polar solvent, such as water, a $C_{1-4}$alkanol (for example methanol or ethanol) or acetic acid. In such cases, the corresponding mineral acid salt of the compound of formula (I) wherein $R^1$ is hydroxy may be conveniently isolated.

Alternatively, base conditions may be used, for example lithium, sodium or potassium hydroxide, conveniently in a suitable solvent or diluent such as an aqueous $C_{1-4}$alkanol at a temperature in the range, for example, 10° to 110°C; or an alkali halide for example lithium chloride in a polar solvent such as dimethylsulphoxide. As yet further alternatives, when $-COR^2$ is t-butoxycarbonyl, the decomposition may be carried out, for example, by

thermolysis at a temperature in the range, for example, 100 to 220°C, alone or in the presence of a suitable diluent such as diphenyl ether.

The compounds of the formula (VI) may be prepared by methods analogous to those described hereinabove for the compounds of the formula (I), with optional protection of the amino function for example with a benzyl group.

Compounds of the formula (VI) also provide a thermogenic effect, in particular compounds of the formula (VI) wherein $R^2$ is $-NH_2$. Accordingly in another aspect the present invention provides compounds of the formula (VI), their use in methods of therapy (in particular in the treatment of obesity and related conditions) and pharmaceutical compositions containing them. The compounds of the formula (VI) are used and formulated in a manner analogous to that described for the compounds of the formula (I).

In the deprotection of a compound of the formula (IX) $R^3$ may for example be an acetal such as 2-methyl-1,3-dioxolan-2-yl or 2-ethyl-1,3-dioxolan-2-yl. Such groups may be readily converted to a group $-COR^1$ wherein $R^1$ is alkyl by acid hydrolysis. The compounds of the formula (IX) may be prepared by methods analogous to those described herein for the preparation of the compounds of the formula (I).

Certain of the compounds of the formulae (IV) and (VIII) are novel. Therefore, in another aspect the present invention provides a compound of the formula (XIV):

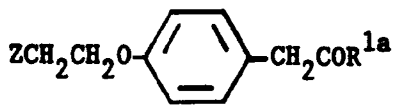

(XIV)

wherein Z is amino, protected amino or a leaving group such as halo or sulphonyloxy and $R^{1a}$ is $C_{1-10}$alkyl.

In vivo hydrolysable esters may be prepared in conventional manner for example by reacting the carboxy group with an alcohol. It will also be realised by the skilled man that groups $R^2$ and $R^3$ in formulae (VI) and (IX) respectively, may be in vivo hydrolysable.

Pharmaceutically acceptable salts may be prepared by reacting the compound of the formula (I) with the appropriate acid in conventional manner. Alternatively when a hydrogen halide salt is required, it may conveniently be obtained by hydrogenation of the free base together with a stoichiometric amount of the corresponding benzyl halide.

The following biological test methods, data and Examples serve to illustrate this invention.

The thermogenic effects of the compound of the formula (I) may be demonstrated using one or more of the following standard tests:-

(a) Rats are cold adapted by being placed in a cold environment (4°C) for 10 days in order to increase their capacity for thermogenesis. They are then transferred to a thermoneutral environment (29°C). Three hours later the core temperature is measured to determine a base-line reading and the test compound is administered sub-cutaneously or orally as a solution or suspension in 0.45% w/v aqueous sodium chloride, 0.25% w/v Polysorbate 80. After one hour, the core temperature is again measured. In this test, a compound which causes a statistically significant increase in the core temperature of about 0.3°C (or more) at a sub-cutaneous dose of 15 mg/kg (or less) is considered to be significantly active. The test acts as a model for the depressed thermogenesis which occurs during dieting.

(b) Rats are cold adapted at 4°C for 4 days to increase their capacity for thermogenesis. They are then transferred to a warm environment of 23°C for 2 days. On the following day, a test compound is administered sub-cutaneously or orally as described in (a). Animals are sacrificed one hour later and the interscapular, brown adipose tissue (BAT) pad is removed. BAT mitochondria are prepared by differential centrifugation and GDP binding is determined (Holloway et al., International Journal of Obesity, 1984, 8, 295) as a measure of thermogenic activation. Each test includes a control which is dosed with the solution/suspension vehicle only and a positive control which is dosed with isoprenaline (as its sulphate) at 1 mg/kg. Test compounds are routinely dosed at 0.1, 0.3, 1.0, 3.0, and 10 mg/kg and results expressed in terms of the effect on GDP binding produced by isoprenaline. From these results, a dose ($ED_{50}$) necessary to produce 50% of the isoprenaline effect is calculated by linear regression analysis. Compounds are considered active in this test if they cause a significant elevation in GDP binding as compared to controls. This test serves to indicate that the thermogenic effects observed in test (a) are mediated through an increase in effect on BAT rather than by some non-specific or toxic mechanism.

(c) Rats are adapted to a thermoneutral environment (29°C) for 2 weeks in order to decrease their capacity for BAT mediated non-shivering thermogenesis. During the final 3 days the animals are trained to use an

apparatus for measuring heart rate non-invasively via foot-pad electrodes connected to an ECG integrator giving a continuous read-out of heart rate. A test compound is administered sub-cutaneously or orally at the $ED_{50}$ determined in test (b), and heart rate is determined 15-30 minutes after dosing. The procedure is then repeated in subsequent tests using increasing multiples of the $ED_{50}$ determined in test (b) until the heart rate (HR) reaches or exceeds 500 beats per minute, allowing the dose necessary to produce a heart rate of 500 beats per minute ($D_{500}$ dose) to be calculated.

The ratio of $D_{500}$ to $ED_{50}$ in test (b) can be defined as the selectivity index (SI) and provides a measure of the selectivity of the compound for BAT as opposed to the cardiovascular system. Compounds are considered to have significant selectivity which have an SI of >1. Non-selective compounds have an SI of <1 (for example isoprenaline = 0.06).

(d) Rats are cold adapted at 4°C for four days to increase their capacity for thermogenesis. They are then transferred to a warm environment at 23°C for two days. On the following day, the basal metabolic rate of the animals is determined using a close-circuit oxygen consumption apparatus of the type described by Arundel et al., 1984, J. Appl. Physiol. Respirat. Environ. Exercise Physiol., 1984, 57 (5) 1591-1593. The rats are then dosed (orally or sub-cutaneously) with test compound at about (10 mg/kg) as a solution or suspension in 0.45% w/v aqueous sodium chloride, 0.25% w/v Polysorbate 80. Metabolic rate is then determined for at least one hour after dosing. Compounds are considered active in this test if they cause a significant increase in metabolic rate as compared to control animals (Student's t test: p <0.5) dosed only the solution or suspension vehicle.

In the above tests, the compounds of the formula (I) in general produce effects of the following order without producing over toxicity:-

test (a): increase in core temperature of about 0.5°C (or more) following a sub-cutaneous dosage of <15 $mgkg^{-1}$;

test (b): sub-cutaneous or oral $ED_{50}$ for GDP binding in BAT mitochondria of 0.01-10 $mgkg^{-1}$; and

test (c): show an SI of >50.

By way of illustration, the compound described in the accompanying Example 5, produced the following effects in the above tests:-

(b) sub-cutaneous (oral) $ED_{50}$ 0.23$mgkg^{-1}$;

(c) $D_{500}$: >23 $mgkg^{-1}$ (oral); SI >100 (oral).

(d) 6.6 ml $O_2$ min $^{-1}Kg^{.75}$ at 1$mgkg^{-1}$ p.o.

The invention will now be illustrated by the following Examples in which, unless otherwise stated:

a) nuclear magnetic resonance (NMR) spectra were determined at 200MHz in $d_6$-dimethylsulphoxide/$d_4$-acetic acid as solvent unless otherwise stated, using tetramethylsilane (TMS) as an internal standard and are expressed in delta values (parts per million) for protons relative to TMS, using conventional abbreviations to describe signal types.

b) chromatography was performed on Merck Kieselgel (Art 7736) (for Example 1) or Kieselgel (Art 9385; 230-400 Mesh) (for Examples 2 - 13) obtainable from E. Merck, Darmstadt, Federal Republic of Germany.

c) where noted, solutions were taken to pH values as judged by moist indicator paper.

d) evaporations were carried out under reduced pressure using a rotary evaporator.

e) melting-points are uncorrected.

## Example 1

4-[2-(2-Hydroxy-3-phenoxypropylamino)ethoxy]phenylacetic acid.

4-[2-(2-Hydroxy-3-phenoxypropylamino)ethoxy]phenylacetamide (0.3g) was heated on the steam-bath for 4 hours in 2N HCl (10ml). The reaction mixture was cooled and the solid collected by filtration. The solid was crystallised from aqueous acetone to give 4-[2-(2-hydroxy-3-phenoxypropylamino)ethoxy]phenylacetic acid as the hydrochloride (0.18g), m.p. 190°C; microanalysis: found C,59.6; H,6.3; N,3.6; Cl,9.2%; required for $C_{19}H_{24}ClNO_5$: C,59.8; H,6.3; N,3.7; Cl,9.3%; NMR 3.10 (dd,1H, one H of -CH(OH)$C\underline{H_2}$NH); 3.20(m,1H, the other H of -CH(OH)$C\underline{H_2}$NH); 3.40(t,2H,NHC$\underline{H_2}$$CH_2$); 3.51(s,2H,C$\underline{H_2}$COOH); 4.00(m,2H,OC$\underline{H_2}$CH(OH)-); 4.2-4.35(m,3H,-C$\underline{H}$(OH)- and -OC$\underline{H_2}$$CH_2$-); 5.87(d,1H,OH); 6.95-7.30(m,9H, aromatic H); 8.7-9.3(br,2H,$NH_2$).

The starting material was obtained as follows:

A mixture of 4-(2-N-benzylaminoethoxy)phenylacetamide (OLS 2135678) (3.38g), phenylglycidyl ether (1.79g) and ethanol (150ml) was heated under reflux for 18 hours. The mixture was cooled and the solvent evaporated under reduced pressure. The residue was purified by dry, flash column chromatography. Elution with 10% methanol in dichloromethane gave 4-[2-(N-benzyl-N-(2-hydroxy-3-phenoxypropyl)amino)ethoxy]phenylacetamide as an oil (4.98g). This was dissolved in methanol (70ml) and acetic acid (30ml). The solution obtained was hydrogenated in the presence of 10% w/w palladium on carbon (0.5g) at about 20 bar

and 60°C for 48 hours. The mixture was cooled, filtered and the filtrate was evaporated under reduced pressure. The residual oil was dissolved in methanol and heated with a solution of ether saturated with hydrogen chloride. The precipitated solid was crystallised from methanol to give 4-[2-(2-hydroxy-3- phenoxypropylamino)ethoxy]phenylacetamide hydrochloride (4.3g), m.p. 249°C; microanalysis: found C, 60.0; H, 6.6; N, 7.4; Cl, 9.3%; required for $C_{19}H_{25}ClN_2O_4$: C, 59.9; H, 6.6; N, 7.4; Cl, 9.3%.

**Example 2**

1-[4-(2-(2-Hydroxy-3-phenoxypropylamino)ethoxy)phenyl]propan-2-one.

4-(2-Phthalimidoethoxy)phenylpropan-2-one (10g) in ethanol (40ml) was treated at 0°C with 40 wt% aqueous methylamine solution (10ml). The reaction mixture was stirred for 45 minutes at room temperature, treated with water (20ml) and stirring was continued for a further 45 minutes. Ethanol was evaporated under reduced pressure and the residue was basified with 30% NaOH. Water (100ml) was added and the mixture extracted with ethyl acetate (3 x 100ml). The combined organic extracts were dried, evaporated to dryness and subjected to chromatography using methanol:ethyl acetate (30:70) as eluant.

The desired fractions were combined, collected, evaporated and heated for 3 hours in propan-1-ol (100ml) with an equimolar quantity of phenylglycidyl ether. The reaction solution was evaporated to dryness under reduced pressure and the residue was subjected to chromatography as before using 5% methanol in dichloromethane as eluant. The appropriate fraction was crystallised from ethanol 1-[4-(2-(2-hydroxy-3-phenoxypropylamino)ethoxy)phenyl]-propan-2-one m.p. 105-106°C; microanalysis: found C, 69.7; H, 7.2; N, 4.2%; required for $C_{20}H_{25}NO_4$:C, 70.0; H, 7.3; N, 4.1%; NMR ($CDCl_3$) 2.13(s,3H, $CH_3$), 2.80-3.10(m,4H, $CH_2NCH_2$), 3.62(s,2H,$CH_2$), 3.97-4.12(m,5H, $OC\underline{H}_2$, $C\underline{H}_2O$ and $C\underline{H}OH$), 6.83-7.32(m,9H, aromatic H).

The starting material was obtained as follows:

A solution of 4-hydroxyphenylpropan-2-one (22.5g), N-(2-hydroxyethyl)phthalimide (28.6g) and triphenylphosphine (38.5g) in tetrahydrofuran (1L) was treated dropwise at -5°C with diethyl azodicarboxylate (23ml). The reaction mixture was stirred at room temperature overnight, evaporated to dryness and treated with ether (500ml). The solution was kept overnight at about 4°C and the solid was collected and recrystallised from ethanol to give 4-(2-phthalimidoethoxy)phenylpropan-2-one, m.p. 116-117°C; microanalysis: found C, 70.5; H, 5.4; N, 4.4%; required for $C_{19}H_{17}NO_4$: C, 70.6; H, 5.2; N, 4.3%.

**Example 3**

1-[4-(2-(2-Hydroxy-3-phenoxypropylamino)ethoxy)phenyl]propan-2-one.

1-[4-((2-Methyl-1,3-dioxolan-2-yl)methyl)phenoxy]-2-(p-toluenesulphonyloxy)ethane (1g) and 1-phenoxy-3-aminopropan-2-ol (0.4g) were heated at 65°C for 12 hours in dimethylsulphoxide (10ml). The cooled reaction mixture was treated with ice-cold 2N HCl (50ml) at ambient temperature for 1 hour. The mixture was basified with solid sodium carbonate and extracted with ether (2 x 50ml). The combined ether extracts were dried, evaporated to dryness and the residue treated with a small volume of acetone to yield a solid, identical in all respects to the product of Example 2.

The starting-material was obtained as follows:

A solution of 2-[4-[(2-methyl-1,3-dioxolan-2-yl)methyl]phenoxy]ethanol (EP-A-70133) (30g) in anhydrous pyridine (500ml) was treated at 0°C with p-toluenesulphonyl chloride (25g) in portions. The reaction mixture was stirred at 0°C for 2 hours and at ambient temperature for 2 hours. Most of the pyridine was evaporated under reduced pressure. Water (1.5L) was added, followed by sufficient saturated sodium hydrogen carbonate solution to take the pH of the solution to approximately 9. The mixture was extracted with ether (3 x 500ml) and the combined organic extracts were washed with N HCl (100ml) and with saturated sodium hydrogen carbonate solution (several times) until the extracts were alkaline. The ether solution was dried and evaporated to dryness to yield 1-[4-((2-methyl-1,3-dioxolan-2-yl)methyl)phenoxy]-2-(p-toluenesulphonyloxy)ethane, m.p. 80°C (from ethanol); microanalysis: found: C, 61.0; H, 6.1%; required for $C_{20}H_{24}O_6S$: C, 61.2; H, 6.1%.

**Example 4**

1-[4-(2-(2-Hydroxy-3-phenoxypropylamino)ethoxy)phenyl]butan-2-one

1-[2-(4-(2-Ethyl-1,3-dioxolan-2-ylmethyl)phenoxy)ethylamino] -3-phenoxypropan-2-ol (2.0g) in a mixture of methanol (38ml) and concentrated hydrochloric acid (38ml) was stirred at 65°C for 1 1/2 hours. The reaction mixture was cooled, poured into water (120ml), basified with sodium hydroxide solution and extracted into ethyl acetate (3 x 50ml). The ethyl acetate extracts were washed with brine (2 x 50ml), dried and the solvent removed under reduced pressure. The residual solid was converted to the hydrochloride and the salt crystallised from

ethanol to yield white crystals of the title compound in the form of the hydrochloride (1.70g); m.p. 167-169°C; microanalysis: found C, 63.9; H, 7.2; N, 3.4%; required for $C_{21}H_{28}ClNO_4$, C,64.0; H, 7.1; N, 3.6%; NMR 0.93(t,3H,$COCH_2CH_3$), 2.48(q,2H,$COCH_2CH_3$), 3.06-3.47(m,4H,$CH_2NHCH_2$), 3.68(s,2H,$ArCH_2CO$), 3.92-4.08(m,2H,$PhOCH_2CH$), 4.22-4.38(m,3H,CHOH and $CH_2CH_2OAr$), 6.88-7.35(m,9H, aromatic H).

The starting material was prepared as follows:

a) 1-(4-Hydroxyphenyl)butan-2-one was prepared by the action of diethylcadmium on 4-methoxyphenylacetyl chloride in toluene followed by demethylation in a mixture of hydrobromic acid and acetic acid as described by W R Biggerstaff et al. J. Med. Chem. 1964,7, 110-113.

b) A solution of 1-(4-hydroxyphenyl)butan-2-one (10.0g), 1,2-ethanediol (7.5ml) and p-toluenesulphonic acid (100mg) in toluene (200ml) was stirred under reflux in a Dean and Stark apparatus for 2 hours in an atmosphere of argon. The reaction mixture was cooled, washed with 5% aqueous sodium bicarbonate (150ml), washed with water (2 x 100ml), washed with brine (100ml) and dried. Toluene was removed under reduced pressure and the residual oil triturated with hexane to give a light brown solid which was crystallised from toluene to give 4-[(2-ethyl-1,3-dioxolan-2-yl)methyl]phenol as white crystals (10.2g) m.p. 88-90°C; microanalysis: found C, 69,3; H, 7.8%; required for $C_{12}H_{16}O_3$: C, 69.2; H, 7.7%.

c) Diethyl azodicarboxylate (9.7ml) was added dropwise to a stirred solution of 4-[(2-ethyl-1,3-dioxolan-2-yl)methyl]phenol (10.0g), N-t-butoxycarbonylaminoethanol (13.3g) and triphenylphosphine (16.3g) in anhydrous tetrahydrofuran (300ml), at ice-bath temperature, under argon. The reaction mixture was left for 64 hours at 20°C and then the solvent was removed under reduced pressure. The residue was dissolved in ether (600ml) and the ethereal solution washed with 2N aqueous sodium hydroxide (2 x 150ml), water (2 x 100ml) and saturated brine (100ml). The aqueous washings were in turn extracted with ether (100ml). The ethereal solutions were combined, dried, and the ether removed under reduced pressure. The residue was purified by chromatography using 35% ethyl acetate in hexane as eluant. Combining the appropriate fractions and crystallisation from hexane yielded 1-t-butoxycarbonylamino-2-[4-(2-ethyl-1,3-dioxolan-2-yl)methyl]-phenoxy]ethane as white crystals (11.1g) m.p 64-66°C; microanalysis: found C, 65.4; H, 8.0; N, 4.2%; required for $C_{19}H_{29}NO_5$: C, 65.0; H, 8.3; N 4.0%.

d) The product from c) above (11.0g) was added to a stirred suspension of sodium hydride (1.50g of a 50% dispersion in mineral oil) in dry dimethylformamide (220ml) under argon. The reaction mixture was stirred at 50°C for 1 1/2 hours. The mixture was cooled to 0°C, phenylglycidyl ether (4.3ml) was added and the reaction mixture then stirred at 50°C for 1 3/4 hours. The reaction mixture was cooled, poured into water (1.2L) and the product extracted into ethyl acetate (400ml, 250ml, 200ml). The combined ethyl acetate extracts were washed with water (500ml), dried and the solvent removed under reduced pressure. The residue was purified by chromatography using 60% ethyl acetate in hexane as eluant. The appropriate fractions were combined, evaporated and the residue triturated with hexane to give a solid which was crystallised from cyclohexane to give 3-[2(4-(2-ethyl-1,3-dioxolan-2-ylmethyl)phenoxy)ethyl]-5-(phenoxymethyl)oxazolidin-2-one as white crystals (4.0g), m.p. 88-90°C; microanalysis: found C, 67.4; H, 7.0; N, 3.2%; required for $C_{24}H_{29}NO_6$: C, 67.4; H, 6.8; N, 3.3%.

e) A solution of product from d) above (4.0g) in a mixture of 2N aqueous sodium hydroxide (12ml) and ethanol (60ml) was heated under reflux under argon for 8 hours. The reaction mixture was cooled, poured into water (100ml) and the product extracted into dichloromethane (3 x 60ml). The combined extracts were washed with brine (60ml), dried, and the solvent removed under reduced pressure. The residual solid was crystallised from ethanol to yield 1-[2-(4-(2-ethyl-1,3-dioxolan-2-ylmethyl)phenoxy)ethylamino]-3-phenoxypropan-2-ol as white crystals (3.0g) m.p. 101-103°C; microanalysis: found C, 69.1; H, 7.8; N, 3.4%; required for $C_{23}H_{31}NO_5$: C, 68.8; H,7.7; N, 3.5%.

## Examples 5 and 6

In a manner similar to that of Example 4, the following compounds as the hydrochloride salts were prepared from 1-(4-hydroxyphenyl)pentan-2-one and 1-(4-hydroxyphenyl)-3-methylbutan-2-one respectively.

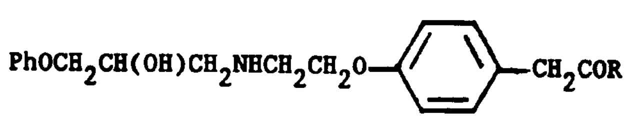

| Example | R | m.p. (solvent) | Microanalysis/NMR |
|---|---|---|---|
| 5 | $-CH_2CH_2CH_3$ | 165-7°C (ethanol) | Found: C, 64.9; H, 7.5; N, 3.3%; required for $C_{22}H_{30}ClNO_4$: C, 64.8; H, 7.4; N, 3.4%; NMR 0.83(t,3H,$COCH_2CH_2C\underline{H}_3$), 1.49(sextet, 2H, $COCH_2C\underline{H}_2CH_3$), 2.44(t,2H, $COC\underline{H}_2CH_2CH_3$), 3.11-3.50(m,4H, $C\underline{H}_2NHC\underline{H}_2$), 3.67(s,2H, $ArC\underline{H}_2CO$-), 3.95-4.09(m,2H,$PhOC\underline{H}_2CH$-), 4.22-4.38(m,3H,C$\underline{H}$OH and $CH_2C\underline{H}_2OAr$), 6.90-7.34(m,9H,aromatic H). |

| Example | R | m.p. (solvent) | Microanalysis/NMR |
|---|---|---|---|
| 6 | $-CH(CH_3)_2$ | 175-7°C (ethanol) | Found: C, 64.9; H, 7.4; N, 3.3%; required for $C_{22}H_{30}ClNO_4$: C, 64.8; H, 7.4; N, 3.4%; NMR 1.05(d,6H,$COCH(C\underline{H}_3)_2$), 2.74(septet, 1H, $COC\underline{H}(CH_3)_2$), 3.18-3.54(m,4H,$C\underline{H}_2NHC\underline{H}_2$), 3.74(s,2H,$ArC\underline{H}_2CO$), 3.98-4.11(m,2H,$PhOC\underline{H}_2CH$-), 4.29-4.40(m,3H,C$\underline{H}$OH and $CH_2C\underline{H}_2OAr$), 6.90-7.34(m,9H, aromatic H). |

For convenience the scheme for the preparation of the compounds of Examples 4-6 is set out below (wherein R is ethyl, n-propyl or isopropyl).

Scheme 1

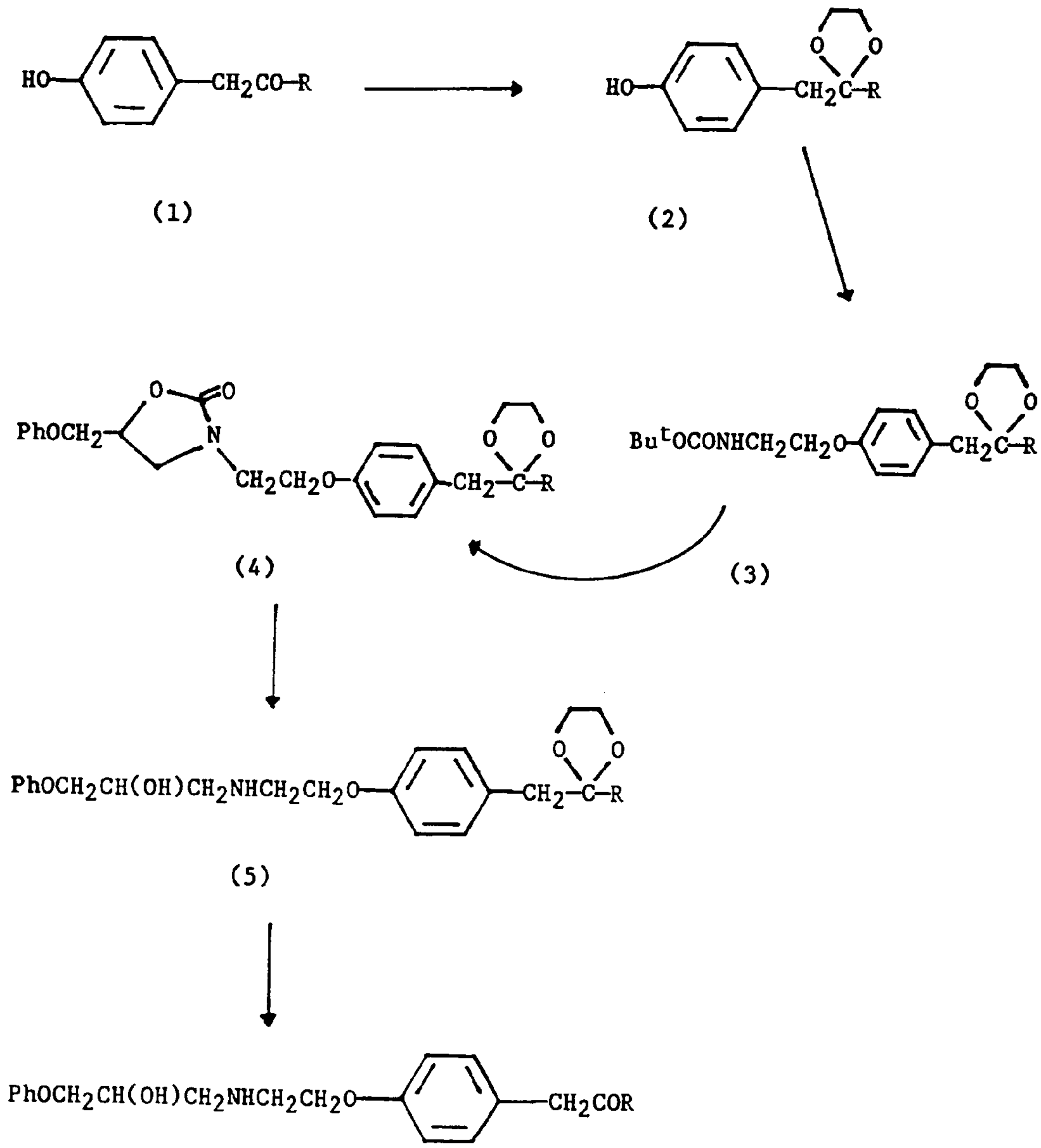

(1)

(2)

(4)

(3)

(5)

(6)

Footnotes to scheme:

A. 1-(4-Hydroxyphenyl)pentan-2-one (compound(1): R = $-CH_2CH_2CH_3$) was prepared as follows:

1-Bromopropane (33.2ml) in anhydrous ether (100ml) was added dropwise over 30 minutes to a stirred suspension of lithium slices (6.0g; 0.86 gm atoms) in ether (150ml) under argon, maintaining the internal temperature at 0° to -10°C. The reaction mixture was stirred for 1 1/2 hours at 0-5°C and added to a stirred solution of 4-hydroxyphenylacetic acid (5.6g) in anhydrous tetrahydrofuran (300ml) at 0 - 10°C under argon, leaving behind any unreacted lithium. The reaction mixture was stirred for 40 hours at 22°C, then cooled in an ice-bath and chlorotrimethylsilane (50ml) was added over 15 minutes. The temperature was allowed to rise to 22°C. and the reaction mixture was poured into 1N hydrochloric acid (600ml) and stirred for 1/2 hour. The product was extracted into ethyl acetate (3 x 200ml) and the combined extracts washed with aqueous sodium bicarbonate (2 x 300ml), washed with brine (100ml), dried and the solvent removed under reduced pressure. The procedure was repeated three times on the same scale and the combined crude ketone was purified by chromatography using 22% ethyl acetate in hexane as eluant. The appropriate fractions were combined and evaporated to yield 1-(4-hydroxyphenyl)pentan-2-one (3.4g) as a yellow oil. (A similar procedure for the preparation of methyl ketones is described by G M Rubottom and Chong-Wan Kim, J.O.C. 1983, 48, 1550-1552).

B. 1-(4-Hydroxyphenyl)-3-methylbutan-2-one (compound (1): R = $-CH(CH_3)_2$) was prepared as follows:

A solution of isopropyl magnesium bromide (prepared from magnesium (1.75g) and 2-bromopropane (8.9g)) in dry tetrahydrofuran (100ml) was added dropwise over 30 minutes to a stirred solution of 4-methoxyphenylacetyl chloride (26.6g) in tetrahydrofuran (75ml) under argon, maintaining the internal temperature at -65° to -70°C. The reaction mixture was allowed to warm up to 20°C over 1 hour and then poured into water (150ml). The product was extracted into ether (2 x 70ml). The ether extracts were washed with 1N aqueous sodium

hydroxide (2 x 70ml), brine (70ml), dried, and the solvent removed under reduced pressure. The residue was purified by chromatography using 10% ethyl acetate in hexane as eluant. The appropriate fractions were combined and the solvent removed under reduced pressure to yield 1-(4-methoxyphenyl)-3-methylbutan-2-one (2.5g) an oil.

1-(4-Methoxyphenyl)-3-methylbutan-2-one (10.2g) was added to a stirred melt of pyridine hydrochloride (36ml) at 140°C under argon. The reaction mixture was heated to 220°C for 2 hours. The mixture was cooled, water (400ml) was added and the product extracted into ether (3 x 150ml). The combined ethereal extracts were washed with brine (100ml), dried, and the solvent removed under reduced pressure. The residual oil was purified by chromatography using 25% ethyl acetate in hexane as eluant. The appropriate fractions were combined to give 1-(4-hydroxyphenyl)-3-methylbutan-2-one (7.9g) as an oil.

C. Physical Data for Intermediates in the preparation of the compounds of Examples 5 and 6.

| Example 5 | m.p. (solvent) | Microanalysis |
|---|---|---|
| Compound (2) | oil | |
| Compound (3) | 80-2°C (cyclohexane) | Found C, 65.6; H, 8.6; N, 3.8%; required for $C_{20}H_{31}NO_5$:C, 65.8; H, 8.5; N, 3.8% |
| Compound (4) | 90-1°C (cyclohexane) | Found C, 67.0; H, 7.1; N, 3.3%; required for $C_{25}H_{31}NO_6$ $1/2H_2O$:C, 66.7; H, 7.1; N,3.1% |
| Compound (5) | 104-6°C (ethanol) | Found C, 69.4; H, 8.0; N, 3.2%; required for $C_{24}N_{33}NO_5$:C, 69.4; H, 8.0; N, 3.4% |

| Example 6 | m.p. (solvent) | Microanalysis |
|---|---|---|
| Compound (2) | 84-6°C (cyclohexane) | Found C, 70.8; H, 8.5%; required for $C_{13}H_{18}O_3$ C, 70.3; H, 8.1% |
| Compound (3) | 113-5°C (cyclohexane) | Found C, 65.7; H, 8.5; N, 3.7%; required for $C_{20}H_{31}NO_5$ C, 65.8; H, 8.5; N, 3.8% |
| Compound (4) | 128-9°C (toluene) | Found C, 67.6; H, 6.8; N, 3.1%; required for $C_{25}H_{31}NO_6$ C, 68.0; H, 7.0; N, 3.2% |
| Compound (5) | 98-100°C (ethanol) | Found C, 69.2; H, 7.9; N, 3.3%; required for $C_{24}H_{33}NO_5$ C, 69.4; H, 8.0; N, 3.4% |

## Example 7

1-[4-(2-(2-Hydroxy-3-phenoxypropylamino)ethoxy)phenyl]butan-2-one.

A solution of 3-[2-(4-(2-oxobutyl)phenoxy)ethyl]-5-(phenoxymethyl)oxazolidin-2-one (200mg) in hot ethanol (4ml) was treated with 2N aqueous sodium hydroxide (0.75ml) and the reaction mixture was stirred under reflux for 4 hours under argon. The reaction mixture was cooled, poured into water (20ml) and the product was extracted into dichloromethane (3 x 20ml). The dichloromethane extracts were washed with brine (20ml), dried, and the solvent was removed under reduced pressure. The residue was converted to the hydrochloride and crystallised from ethanol to yield the title compound in the form of the hydrochloride (150mg); m.p. 166-8°C.

The starting material was prepared as follows:

a) A solution of N-t-butoxycarbonylaminoethanol (2.0g), dihydropyran (1.7ml) and pyridinium p-toluenesulphonate (310mg) in anhydrous dichloromethane (90ml) was stirred at room temperature for 2 1/2 hours. Ether (150ml) was added, the solution was washed with water (2 x 50ml), dried, and the solvent removed under reduced pressure to give 2-(2-(N-t-butoxycarbonylamino)ethoxy)tetrahydropyran (3.0g) as a yellow oil.

b) The product from a) above (2.9g) was added to a stirred suspension of sodium hydride (570mg of a 50% dispersion in mineral oil, 0.012 moles) in anhydrous dimethylformamide (100ml) under argon. The reaction mixture was stirred at 55°C for 1 1/2 hours. The mixture was cooled to 0°C, phenylglycidyl ether (1.6ml) was added and the reaction mixture then stirred at 20°C for 2 1/2 hours. The reaction mixture was poured into water (500ml) and the product extracted into ethyl acetate (350ml, 100ml, 100ml). The combined ethyl acetate extracts were washed with water (200ml), dried, and the solvent removed under reduced pressure. The residue was subjected to chromatography using 60% ethyl acetate in hexane as eluant. The appropriate fractions were combined to yield 5-phenoxymethyl-3-(2-(tetrahydropyran-2-yloxy)ethyl)oxazolidin-2-one (1.0g) m.p. 78-80°C (cyclohexane); microanalysis: found C, 63.3; H, 7.2; N, 4.3%; required for $C_{17}H_{23}NO_5$: C, 63.6; H, 7.2; N, 4.4%.

c) A solution of the product of b) above (0.88g) and pyridinium p-toluenesulphonate (70mg) in a mixture of water (1.0ml) and ethanol (24ml) was stirred at 65°C for 4 hours. Ethanol was removed under reduced pressure and the residue partitioned between water (30ml) and ethyl acetate (30ml). The organic layer was separated and the aqueous layer re-extracted with ethyl acetate. The ethyl acetate extracts were washed with water (20ml), dried, and the solvent removed under reduced pressure. Trituration of the residue with ether followed by crystallisation from propan-2-ol yielded 5-phenoxymethyl-3-(2- hydroxyethyl)oxazolidin-2-one (0.52g) m.p 89-91°C; microanalysis: found C, 60.8; H, 6.3; N, 5.8%; required for $C_{12}H_{15}NO_4$: C, 60.8; H, 6.3; N, 5.9%.

d) Diethyl azodicarboxylate (0.33ml) was added dropwise to a stirred solution of 1-(4-hydroxyphenyl)butan-2-one (0.35g), part of the product from c) above (0.50g) and triphenylphosphine (0.55g) in anhydrous tetrahydrofuran (13ml), at ice-bath temperature, under argon. The reaction mixture was left for 100 hours at 20°C and then the solvent removed under reduced pressure. The residue was dissolved in ethyl acetate and the solution seeded with 1,2-dicarbethoxyhydrazine. The solution was allowed to stand for 2 hours,

the solid was collected and the filtrate was subjected to chromatography using 50% ethyl acetate in hexane as eluant. The appropriate fractions were combined, evaporated, and the residue was crystallised from ethyl acetate/cyclohexane to give 3-[2-(4-(2-oxobutyl)phenoxy)ethyl]-5-(phenoxymethyl)oxazolidin-2-one (280mg); m.p 67-69°C; microanalysis found C, 68.9; H, 6.6; N, 3.7%; required for $C_{22}H_{25}NO_5$: C, 68.9; H, 6.5; N, 3.7%.

**Examples 8 - 12**

In a manner similar to that of Example 7, the following compounds as the hydrochloride salts were prepared from 1-(4-hydroxyphenyl)hexane-2-one, 1-(4-hydroxyphenyl)-4-methylpentan-2-one, 1-(4-hydroxyphenyl)heptan-2-one, 1-(4-hydroxyphenyl)octan-2-one and 1-(4-hydroxyphenyl)nonan-2-one respectively.

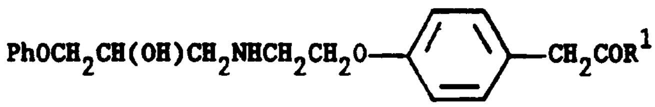

| Example | $R^1$ | m.p. (solvent) | Microanalysis/NMR |
|---|---|---|---|
| 8 | $-(CH_2)_3CH_3$ | 173-5°C (ethanol) | Found: C,65.1; H,7.6; N,3.3%; required for $C_{23}H_{32}ClNO_4$: C,65.5; H,7.6; N, 3.3%; NMR 0.84(t,3H, $COCH_2CH_2CH_2C\underline{H}_3$) 1.23(sextet, 2H, $COCH_2CH_2C\underline{H}_2CH_3$); 1.46(quintet, 2H, $COCH_2C\underline{H}_2CH_2CH_3$); 2.46(t,2H, $COC\underline{H}_2CH_2CH_2CH_3$); 3.09-3.47(m,4H, $C\underline{H}_2NHC\underline{H}_2$); 3.68(s,2H,$ArC\underline{H}_2CO$); 3.95-4.09(m,2H,$PhOC\underline{H}_2CH$); 4.22-4.36(m,3H,$C\underline{H}OH$ and $CH_2C\underline{H}_2OAr$); 6.90-7.36(m,9H, aromatic H). |
| 9 | $CH_2CH(CH_3)_2$ | 176-8°C (ethanol) | Found: C,65.1; H,7.5; N, 3.1%; required for $C_{23}H_{32}ClNO_4$; C, 65.5; H,7.6; N, 3.3%; NMR 0.84(d,6H, $COCH_2CH(C\underline{H}_3)_2$); 2.04(m,1H, $COCH_2C\underline{H}(CH_3)_2$); 2.35(d,2H,$COC\underline{H}_2CH(CH_3)_2$); 3.10-3.50(m,4H,$C\underline{H}_2NHC\underline{H}_2$); 3.66(s,2H $ArC\underline{H}_2CO$); 3.95-4.10(m,2H,$PhOC\underline{H}_2CH$); 4.22-4.36(m,3H,$C\underline{H}OH$ and $CH_2C\underline{H}_2OAr$); 6.92-7.35(m,9H, aromatic H). |

| Example | $R^1$ | m.p. (solvent) | Microanalysis/NMR |
|---|---|---|---|
| 10 | $(CH_2)_4CH_3$ | 172-4°C (ethanol) | Found: C,65.8; H,7.8; N,3.4%; required for $C_{24}H_{34}ClNO_4$: C,66.1; H,7.8; N,3.2%; NMR 0.84(t,3H, $CH_2CH_2CH_2CH_2C\underline{H}_3$); 1.10-1.35(m,4H, $CH_2CH_2C\underline{H}_2C\underline{H}_2CH_3$); 1.48(quintet, 2H, $CH_2C\underline{H}_2CH_2CH_2CH_3$); 2.46(t,2H, $COC\underline{H}_2CH_2CH_2CH_2CH_3$); 3.12-3.50(m,4H,$C\underline{H}_2NHC\underline{H}_2$); 3.67(s,2H, $ArC\underline{H}_2CO$); 3.95-4.10(m,2H,$PhOC\underline{H}_2CH$); 4.24-4.39(m,3H,$C\underline{H}OH$ and $CH_2C\underline{H}_2OAr$); 6.90-7.36(m,9H, aromatic H) |
| 11 | $(CH_2)_5CH_3$ | 172-4°C (ethanol) | Found: C,66.8; H, 8.3; N, 3.0%; required for $C_{25}H_{36}ClNO_4$: C,66.7; H,8.0; N, 3.1%; NMR 0.85(t,3H, $CH_2CH_2CH_2CH_2CH_2C\underline{H}_3$); 1.15-1.32(m,6H, $CH_2CH_2C\underline{H}_2C\underline{H}_2C\underline{H}_2CH_3$); 1.46(quintet,2H,$CH_2C\underline{H}_2CH_2CH_2CH_2CH_3$); 2.46(t,2H,$COC\underline{H}_2CH_2$); 3.08-3.50(m,4H, $C\underline{H}_2NHC\underline{H}_2$); 3.67(s,2H,$ArC\underline{H}_2CO$); 3.94-4.10(m,2H $PhOC\underline{H}_2CH$); 4.23-4.38(m,3H,$C\underline{H}OH$ and $CH_2C\underline{H}_2OAr$); 6.90-7.36(m,9H, aromatic H) |

| Example | $R^1$ | m.p. (solvent) | Microanalysis/NMR |
|---|---|---|---|
| 12 | $(CH_2)_6CH_3$ | 172-4°C (ethanol) | Found: C,67.3; H,8.6: N,3.4%; required for $C_{26}H_{38}ClNO_4$: C,67.3; H,8.2; N,3.0%; NMR 0.86(t,3H, $CH_2CH_2CH_2CH_2CH_2CH_2C\underline{H}_3$); 1.13-1.33(m,8H, $CH_2CH_2C\underline{H}_2C\underline{H}_2C\underline{H}_2C\underline{H}_2CH_3$); 1.48(quintet, 2H,$CH_2C\underline{H}_2CH_2CH_2CH_2CH_2CH_3$); 2.46(t,2H, $COC\underline{H}_2CH_2CH_2$-) 3.12-3.53(m,4H, $C\underline{H}_2NHC\underline{H}_2$); 3.67(s,2H, $ArC\underline{H}_2CO$); 3.97-4.12(m,2H, $PhOC\underline{H}_2CH$); 4.26-4.39(m,3H, $C\underline{H}OH$ and $CH_2C\underline{H}_2OAr$); 6.90-7.36(m,9H, aromatic H) |

For convenience the scheme for the preparation of the compounds of Examples 7-12 is set out below (wherein R is ethyl, n-butyl, isobutyl, n-pentyl, n-hexyl or n-heptyl.

**Scheme 2**

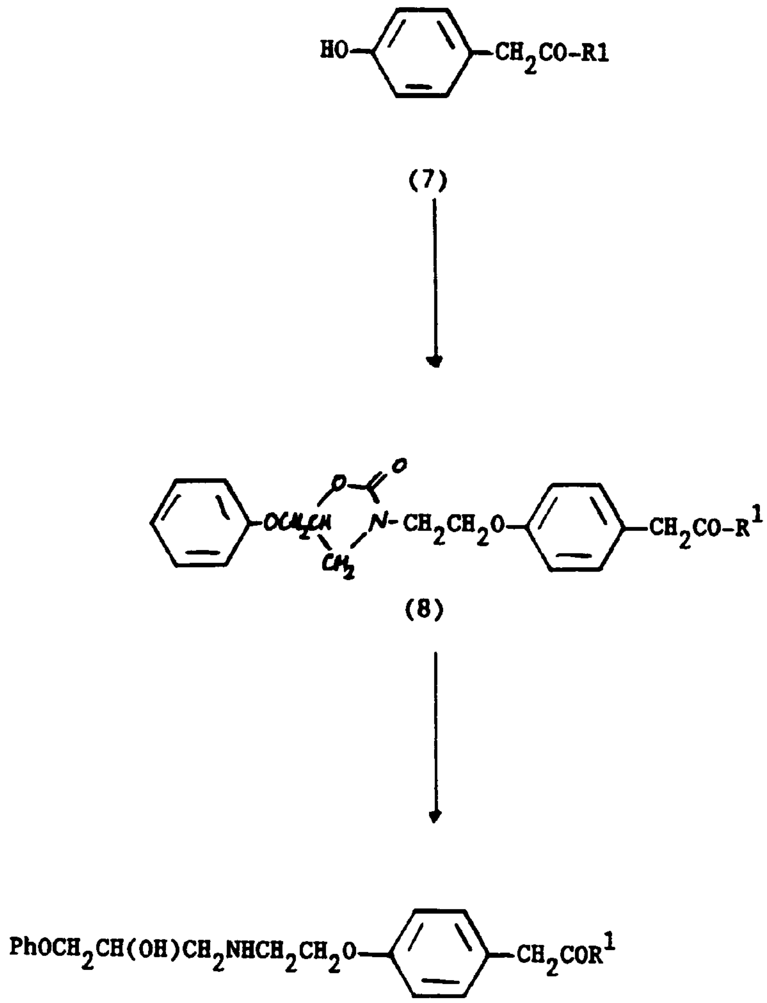

(7)

(8)

(9)

**Footnotes to scheme 2**

D. In a manner similar to that described in footnote A to Scheme 1, 1-(4-hydroxyphenyl)hexan-2-one was prepared from commercially available n-butyllithium.

E. In a manner similar to that described in footnote B to Scheme 1, 1-(4-hydroxyphenyl)-4-methylpentan-2-one, 1-(4-hydroxyphenyl)heptan-2-one, 1-(4-hydroxyphenyl)octan-2-one and 1-(4-hydroxyphenyl)nonan-2-one were prepared from the appropriate alkyl bromide.

F. Physical Data for Intermediates in the preparation of the compounds of Examples 8-12.

| Example 8 | m.p. (solvent) | Microanalysis |
|---|---|---|
| Compound (7) | oil | |
| Compound (8) | 77-9°C (Cyclohexane) | Found C, 69.8; H, 7.1; N, 3.4%; required for $C_{24}H_{29}NO_5$:C, 70.1; H, 7.1; N, 3.4%. |
| **Example 9** | | |
| Compound (7) | oil | |
| Compound (8) | 93-5°C (ethyl acetate/ cyclohexane) | Found C, 70.0; H, 7.2; N, 3.4%; required for $C_{24}H_{29}NO_5$: C, 70.1; H, 7.1; N, 3.4%. |
| **Example 10** | | |
| Compound (7) | Oil | |
| Compound (8) | 76-8°C (ethyl acetate/ cyclohexane) | Found C, 70.8; H, 7.6; N, 3.3%; required for $C_{25}H_{31}NO_5$: C, 70.6; H, 7.3; N, 3.3%. |
| **Example 11** | | |
| Compound (7) | Oil | |
| Compound (8) | 81-3°C (ethyl acetate/ cyclohexane) | Found C, 71.1; H, 7.5; N, 3.1%; required for $C_{26}H_{33}NO_5$: C, 71.1; H, 7.5; N, 3.2%. |

| | | |
|---|---|---|
| **Example 12** | | |
| Compound (7) | 44-6°C (cyclohexane) | Found C, 77.0; H, 9.7%; required for $C_{15}H_{22}O_2$: C, 76.9; H, 9.4%. |
| Compound (8) | 83-5°C (ethyl acetate/ cyclohexane) | Found C, 71.3; H, 7.7; N, 3.0%; required for $C_{27}H_{35}NO_5$: C, 71.5; H, 7.7; N, 3.1%. |

## Example 13

(S)-1-[4-(2-(2-Hydroxy-3-phenoxypropylamino)- ethoxy)phenyl]pentan-2-one.

In a manner similar to that of Example 7, (S)-5-phenoxymethyl-3-(2-hydroxyethyl)oxazolidin-2-one was reacted with 1-(4-hydroxyphenyl)pentan-2-one to give (S)-3-[2-(4-(2-oxopentyl)phenoxy)ethyl]-5-(phenoxymethyl)oxazolidin-2-one which was hydrolysed to the title compound in the form of the hydrochloride; m.p. 166-7°C (ethanol); $[\alpha]_D^{28}$= -11.3° (c = 1.0, methanol); microanalysis: found C, 64.9; H, 7.5; N, 3.7%; required for $C_{22}H_{30}ClNO_4$: C, 64.8; H, 7.4; N, 3.4%; NMR (DMSO-$d_6$) 0.82(t,3H, $CH_2CH_2CH_3$); 1.49(sextet, 2H, $CH_2CH_2CH_3$); 2.44(t,2H,$CH_2CH_2CH_3$); 3.20-3.48(m,4H,$CH_2NHCH_2$); 3.66(s,2H, $ArCH_2CO$); 3.92-4.07(m,2H $PhOCH_2CH$); 4.22-4.38(m,3H, CHOH and $CH_2CH_2OAr$); 5.90(1H,OH); 6.90-7.34(m,9H, aromatic H); 9.13 and 9.35(2H, $NH_2^+$).

The starting material was prepared as follows:

a) (S)-5-Phenoxymethyloxazolidin-2-one was prepared from (S)-phenylglycidyl ether and urea using the procedure described by W. J. Kauffman and J. E. Herweh, J. Org. Chem. 1972, 37, 1842-1845.

b) Sodium hydride (2.2g of a 50% dispersion in mineral oil) was added to a stirred solution of 2-bromoethyl acetate (5ml) and (S)-5-phenoxymethyloxazolidin-2-one (8.0g) in anhydrous dimethylformamide (150ml), under argon, at 20°C. When the effervescence had ceased, the reaction mixture was heated to 45-50°C for 2 hours. The reaction mixture was cooled, poured into water (750ml) and the product extracted into ethyl acetate (3 x 400ml). The combined ethyl acetate extracts were washed with water (300ml), dried, and the solvent removed under reduced pressure. The residue was subjected to chromatography using ethyl acetate in hexane as eluant (gradient elution; 60% to 70% ethyl acetate). The appropriate fractions were combined to yield (S)-5-phenoxymethyl-3-(2-acetoxyethyl)oxazolidin-2-one (4.6g).

c) A solution of the product from b) above (4.5g) and sodium methoxide (1.0g) in methanol (50ml) was stirred at 20°C for 1 hour. Methanol was removed by evaporation and the residue partitioned between ethyl acetate and water. The ethyl acetate layer was separated, dried, and the solvent removed under reduced pressure. The residue was subjected to chromatography using 2% methanol in dichloromethane as eluant. The appropriate fractions were combined to yield (S)-5-phenoxymethyl-3-(2-hydroxyethyl)oxazolidin-2-one (2.2g).

## Claims

**Claims for the following Contracting States : AT, BE, CH, DE, DK, FR, GB, IT, LI, LU, NL, SE**

1. A compound of the formula (I):

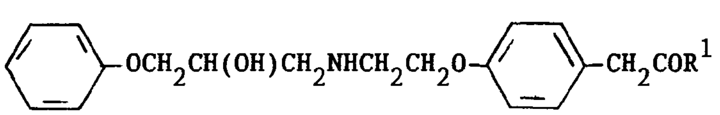

(I)

wherein $R^1$ is hydroxy or $C_{1-10}$alkyl, or an in vivo hydrolysable ester or a pharmaceutically acceptable salt thereof.

2. A compound according to claim 1 wherein $R^1$ is hydroxy.

3. A compound according to claim 1 wherein $R^1$ is $C_{1-6}$alkyl.

4. A compound according to claim 3 wherein $R^1$ is methyl, ethyl, n-propyl, isobutyl, n-butyl or n-hexyl.

5. A compound according to claim 1 which is: 4-[2-(2-hydroxy-3-phenoxypropylamino)ethoxy]phenylacetic acid or (S)-4-[2-(2-hydroxy-3-phenoxypropylamino)ethoxy]phenylacetic acid.

6. A compound according to claim 1 which is:
1-[4-(2-(2-hydroxy-3-phenoxypropylamino)ethoxy)phenyl]propan-2-one,
(S)-1-[4-(2-(2-hydroxy-3-phenoxypropylamino)ethoxy)phenyl]propan-2-one,
(S)-1-[4-(2-(2-hydroxy-3-phenoxypropylamino)ethoxy)phenyl]butan-2-one,
1-[4-(2-(2-hydroxy-3-phenoxypropylamino)ethoxy)phenyl]pentan-2-one,
(S)-1-[4-(2-(2-hydroxy-3-phenoxypropylamino)ethoxy)phenyl]pentan-2-one,
1-[4-(2-(2-hydroxy-3-phenoxypropylamino)ethoxy)phenyl]-3-methylbutan-2-one,
1-[4-(2-(2-hydroxy-3-phenoxypropylamino)ethoxy)phenyl]hexan-2-one,
1-[4-(2-(2-hydroxy-3-phenoxypropylamino)ethoxy)phenyl]-4-methylpentan-2-one,
1-[4-(2-(2-hydroxy-3-phenoxypropylamino)ethoxy)phenyl]heptan-2-one,
1-[4-(2-(2-hydroxy-3-phenoxypropylamino)ethoxy)phenyl]octan-2-one or
1-[4-(2-(2-hydroxy-3-phenoxypropylamino)ethoxy)phenyl]nonan-2-one.

7. A compound according to any one of claims 1 to 6 when in (S) absolute configuration at the -CH(OH)- group.

8. A pharmaceutical composition which comprises a compound according to any one of claims 1 to 6 and a pharmaceutically acceptable carrier.

9. A pharmaceutical composition which comprises a compound according to claim 7 and a pharmaceutically acceptable carrier.

10. A process for preparing a compound of the formula (I) or in vivo hydrolysable ester or pharmaceutically acceptable salt thereof as defined in claim 1 which comprises:
a) reacting a compound of the formula (II) or (III) with a compound of the formula (IV):

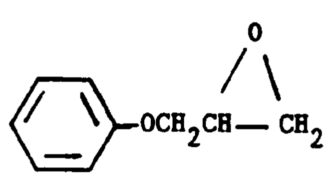

(II)

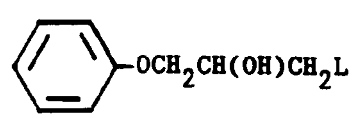

(III)

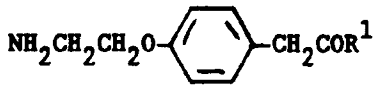

(IV)

wherein $R^1$ is as defined in claim 1 and L is a displaceable group; or

b) hydrolysis of a compound of the formula (V):

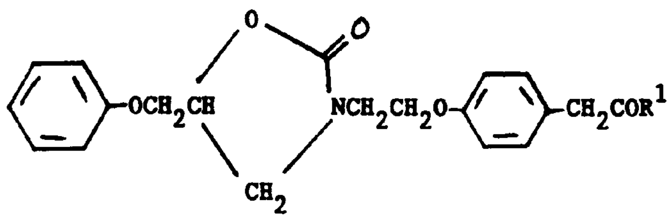

(V)

wherein $R^1$ is as hereinbefore defined; or

c) for compounds of the formula (I) wherein $R^1$ is hydroxy, hydrolysing a compound of the formula (VI):

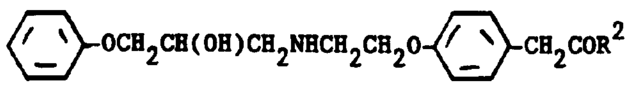

(VI)

wherein $R^2$ is a hydrolysable group;

d) reacting a compound of the formula (VII) with a compound of the formula (VIII):

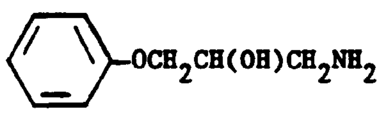

(VII)

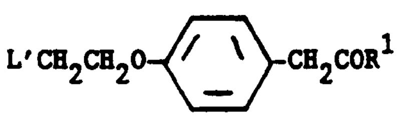

(VIII)

wherein $R^1$ is as hereinbefore defined and L' is a leaving group; or

e) deprotecting a compound of the formula (IX):

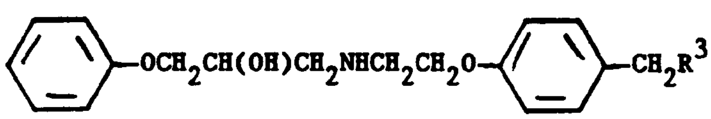

(IX)

wherein $R^1$ is as hereinbefore defined and $R^3$ is a protected derivative of $-COR^1$:

and if necessary thereafter forming an in vivo hydrolysable ester or a pharmaceutically acceptable salt.

**11.** A compound of the formula (VI):

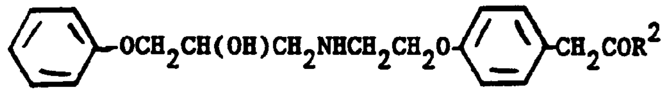

(VI)

wherein $R^2$ is a hydrolysable group.

**12.** A compound according to claim 11 wherein $R^2$ is $C_{1-6}$alkoxy or amino ($-NH_2$).

**13.** A compound of the formula (V):

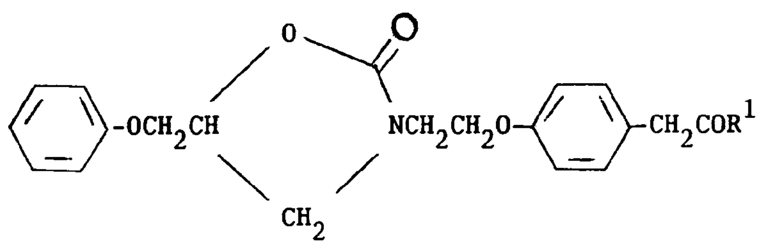

(V)

or of the formula (IX):

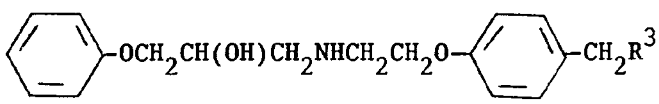

(IX)

wherein $R^1$ is defined as in claim 1 and $R^3$ is a protected derivative of $-COR^1$.

**14.** A compound of the formula (XIV):

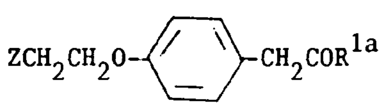

(XIV)

wherein Z is amino, protected amino or a leaving group such as halo or sulphonyloxy and $R^{1a}$ is $C_{1-10}$alkyl with the proviso that when $R^{1a}$ is methyl, Z is not amino or protected amino.

**Claims for the following Contracting State : ES**

**1.** A process for preparing a compound of the formula (I):

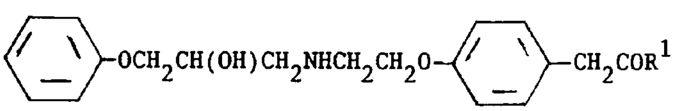

(I)

wherein $R^1$ is hydroxy or $C_{1-10}$alkyl, or an <u>in vivo</u> hydrolysable ester or a pharmaceutically acceptable salt thereof; which process comprises:

a) reacting a compound of the formula (II) or (III) with a compound of the formula (IV):

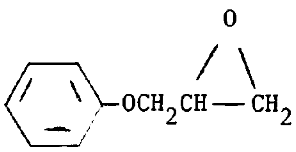

(II)

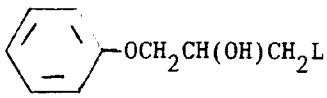

(III)

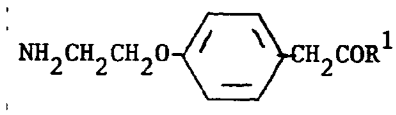

(IV)

wherein $R^1$ is as defined hereinbefore and L is a displaceable group; or

b) hydrolysis of a compound of the formula (V):

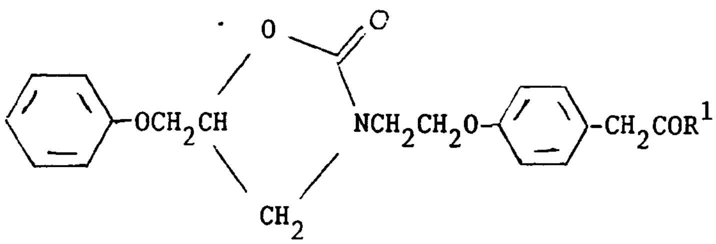

(V)

wherein $R^1$ is as hereinbefore defined; or

c) for compounds of the formula (I) wherein $R^1$ is hydroxy, hydrolysing a compound of the formula (VI):

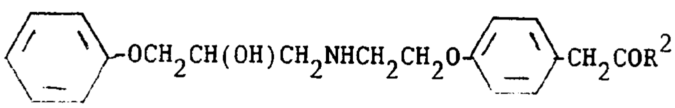

(VI)

wherein $R^2$ is a hydrolysable group;

d) reacting a compound of the formula (VII) with a compound of the formula (VIII):

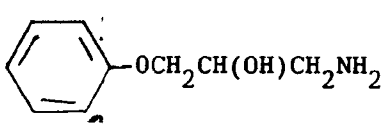

(VII)

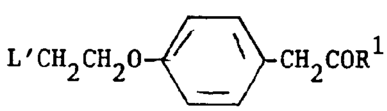

(VIII)

wherein $R^1$ is as hereinbefore defined and L' is a leaving group; or

e) deprotecting a compound of the formula (IX):

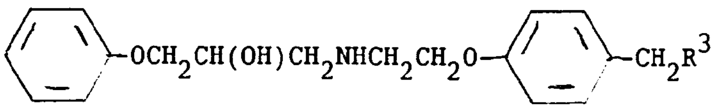 (IX)

wherein $R^1$ is as hereinbefore defined and $R^3$ is a protected derivative of $-COR^1$:
and if necessary thereafter forming an <u>in vivo</u> hydrolysable ester or a pharmaceutically acceptable salt.

2. A process for preparing a compound of the formula (I) according to claim 1 wherein $R^1$ is hydroxy.

3. A process for preparing a compound of the formula (I) according to claim 1 wherein $R^1$ is $C_{1-6}$alkyl.

4. A process for preparing a compound of the formula (I) according to claim 3 wherein $R^1$ is methyl, ethyl, n-propyl, isobutyl or n-butyl or n-hexyl.

5. A process for preparing a compound of the formula (I) according to claim 1 which is:
4-[2-(2-hydroxy-3-phenoxypropylamino)ethoxy]phenylacetic acid or (S)-4-[2-(2-hydroxy-3-phenoxypropylamino)ethoxy]phenylacetic acid.

6. A process for preparing a compound of the formula (I) according to claim 1 which is:
1-[4-(2-(2-hydroxy-3-phenoxypropylamino)ethoxy)phenyl]propan-2-one,
(S)-1-[4-(2-(2-hydroxy-3-phenoxypropylamino)ethoxy)phenyl]propan-2-one,
(S)-1-[4-(2-(2-hydroxy-3-phenoxypropylamino)ethoxy)phenyl]butan-2-one,
1-[4-(2-(2-hydroxy-3-phenoxypropylamino)ethoxy)phenyl]pentan-2-one,
(S)-1-[4-(2-(2-hydroxy-3-phenoxypropylamino)ethoxy)phenyl]pentan-2-one,
1-[4-(2-(2-hydroxy-3-phenoxypropylamino)ethoxy)phenyl]-3-methylbutan-2-one,
1-[4-(2-(2-hydroxy-3-phenoxypropylamino)ethoxy)phenyl]hexan-2-one,
1-[4-(2-(2-hydroxy-3-phenoxypropylamino)ethoxy)phenyl]-4-methylpentan-2-one,
1-[4-(2-(2-hydroxy-3-phenoxypropylamino)ethoxy)phenyl]heptan-2-one,
1-[4-(2-(2-hydroxy-3-phenoxypropylamino)ethoxy)phenyl]octan-2-one or
1-[4-(2-(2-hydroxy-3-phenoxypropylamino)ethoxy)phenyl]nonan-2-one.

7. A process for preparing a compound of the formula (I) according to any one of claims 1 to 6 when in (S) absolute configuration at the -CH(OH)- group.

8. A process for preparing a pharmaceutical composition which comprises bringing into association a compound of the formula (I) according to any one of claims 1 to 7 and a pharmaceutically acceptable carrier.

9. A compound of the formula (VI):

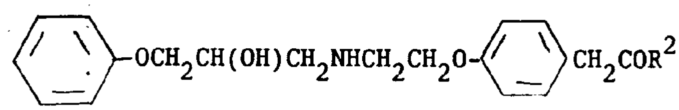 (VI)

wherein $R^2$ is a hydrolysable group.

10. A compound according to claim 9 wherein $R^2$ is $C_{1-6}$alkoxy or amino ($-NH_2$).

11. A compound of the formula (V):

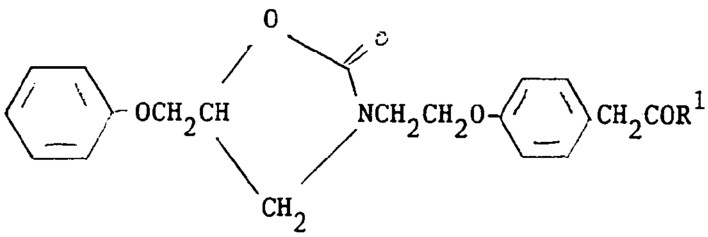

(V)

or of the formula (IX):

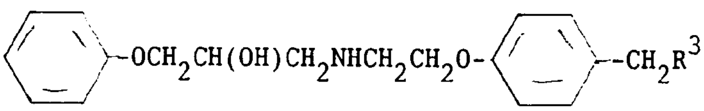

(IX)

wherein $R^1$ is as hereinbefore defined and $R^3$ is a protected derivative of $-COR^1$.

**12.** A compound of the formula (XIV):

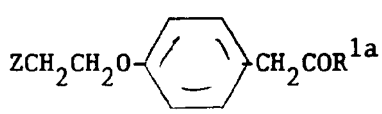

(XIV)

wherein Z is amino, protected amino or a leaving group such as halo or sulphonyloxy and $R^{1a}$ is $C_{1-10}$alkyl with the proviso that when $R^{1a}$ is methyl, Z is not amino or protected amino.

**Patentansprüche**

**Patentansprüche für folgende Vertragsstaaten : AT, BE, CH, DE, DK, FR, GB, IT, LI, LU, NL, SE**

**1.** Verbindungen der Formel I

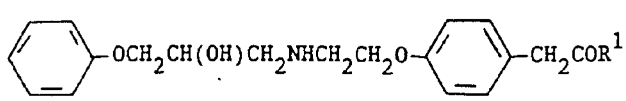

(I)

worin $R^1$ für Hydroxy oder $C_{1-10}$-Alkyl steht, sowie die in vivo hydrolysierbaren Ester und pharmazeutisch zulässigen Salze davon.

**2.** Verbindungen nach Anspruch 1, worin $R^1$ für Hydroxy steht.

**3.** Verbindungen nach Anspruch 1, worin $R^1$ für $C_{1-6}$-Alkyl steht.

**4.** Verbindungen nach Anspruch 3, worin $R^1$ für Methyl, Ethyl, n-Propyl, Isobutyl, n-Butyl oder n-Hexyl steht.

**5.** Verbindungen nach Anspruch 1, nämlich 4-[2-(2-Hydroxy-3-phenoxypropylamino)ethoxy]phenylessigsäure und (S)-4-[2-(2-Hydroxy-3-phenoxypropylamino)ethoxy]phenylessigsäure.

**6.** Verbindungen nach Anspruch 1, nämlich
1-[4-(2-(2-Hydroxy-3-phenoxypropylamino)ethoxy)phenyl]propan-2-on,

(S)-1-[4-(2-(2-Hydroxy-3-phenoxypropylamino)ethoxy]propan-2-on,
(S)-1-[4-(2-(2-Hydroxy-3-phenoxypropylamino)ethoxy)phenyl]butan-2-on,
1-[4-(2-(2-Hydroxy-3-phenoxypropylamino)ethoxy)phenyl]pentan-2-on,
(S)-1-[4-(2-(2-Hydroxy-3-phenoxypropylamino)ethoxy)phenyl]pentan-2-on,
1-[4-(2-(2-Hydroxy-3-phenoxypropylamino)ethoxy)phenyl]-3-methylbutan-2-on,
1-[4-(2-(2-Hydroxy-3-phenoxypropylamino)ethoxy)phenyl]hexan-2-on,
1-[4-(2-(2-Hydroxy-3-phenoxypropylamino)ethoxy)phenyl]-4-methylpentan-2-on,
1-[4-(2-(2-Hydroxy-3-phenoxypropylamino)ethoxy)phenyl]heptan-2-on,
1-[4-(2-(2-Hydroxy-3-phenoxypropylamino)ethoxy)phenyl]octan-2-on und
1-[4-(2-(2-Hydroxy-3-phenoxypropylamino)ethoxy)phenyl]nonan-2-on.

7. Verbindungen nach einem der Ansprüche 1 bis 6, welche an der Gruppe -CH(OH)- die absolute (S)-Konfiguration aufweisen.

8. Pharmazeutische Zusammensetzungen, welche eine Verbindung nach einem der Ansprüche 1 bis 6 sowie einen pharmazeutisch zulässigen Träger enthalten.

9. Pharmazeutische Zusammensetzungen, welche eine Verbindung nach Anspruch 7 sowie einen pharmazeutisch zulässigen Träger enthalten.

10. Verfahren zur Herstellung einer Verbindung der Formel I oder eines in vivo hydrolysierbaren Esters oder pharmazeutisch zulässigen Salzes davon nach Anspruch 1, bei welchem
a) eine Verbindung der Formel II oder der Formel III

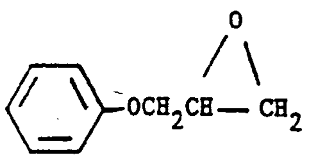

(II)

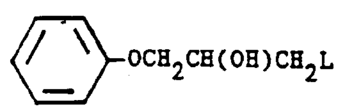

(III)

mit einer Verbindung der Formel IV

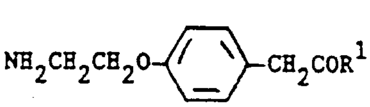

(IV)

umgesetzt wird, wobei $R^1$ die in Anspruch 1 angegebene Bedeutung besitzt und L für eine Austrittsgruppe steht; oder
b) eine Verbindung der Formel V

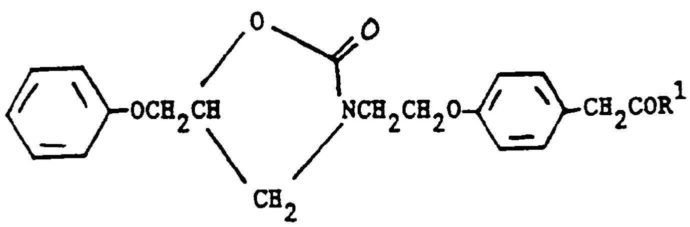

(V)

hydrolysiert wird, wobei $R^1$ die oben angegebene Bedeutung besitzt; oder

c) für Verbindungen der Formel I, worin $R^1$ für Hydroxy steht, eine Verbindung der Formel VI

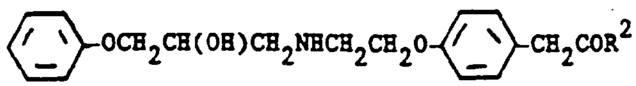

(VI)

hydrolisiert wird, wobei $R^2$ für eine hydrolysierbare Gruppe steht; oder

d) eine Verbindung der Formel VII

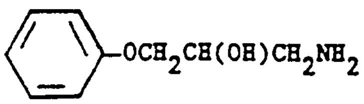

(VII)

mit einer Verbindung der Formel VIII

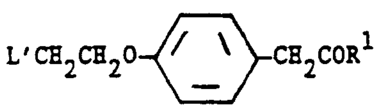

(VIII)

umgesetzt wird, wobei $R^1$ die oben angegebene Bedeutung besitzt und L' für eine Austrittsgruppe steht; oder

e) eine Verbindung der Formel IX

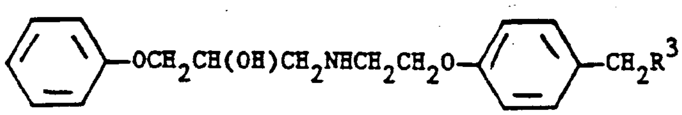

(IX)

worin $R^1$ die oben angegebene Bedeutung besitzt und $R^3$ für ein geschütztes Derivat von $-COR^1$ steht, von der Schutzgruppe befreit wird;

und hierauf gegebenenfalls ein in vivo hydrolysierbarer Ester oder ein pharmazeutisch zulässiges Salz hergestellt wird.

**11.** Verbindungen der Formel VI

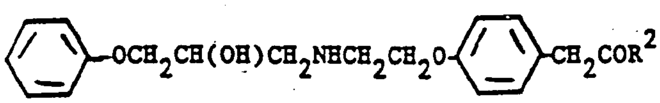

(VI)

worin $R^2$ für eine hydrolysierbare Gruppe steht.

**12.** Verbindungen nach Anspruch 11, worin $R^2$ für $C_{1-6}$-Alkoxy oder Amino ($-NH_2$) steht.

**13.** Verbindungen der Formel V

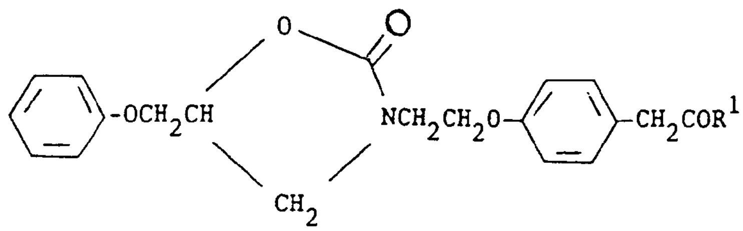

(V)

oder der Formel IX

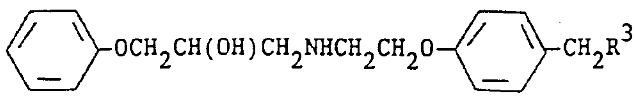

(IX)

worin $R^1$ die in Anspruch 1 angegebene Bedeutung besitzt und $R^3$ für ein geschütztes Derivat von $-COR^1$ steht.

**14.** Verbindungen der Formel XIV

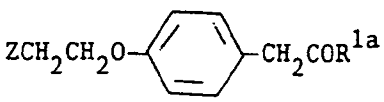

(XIV)

worin Z für Amino, geschütztes Amino oder eine Austrittsgruppe, wie z. B. Halogeno oder Sulfophenyloxy, steht und $R^{1a}$ für $C_{1-10}$-Alkyl steht, mit der Maßgabe, daß, wenn $R^{1a}$ für Methyl steht, Z nicht Amino oder geschütztes Amino bedeutet.

**Patentansprüche für folgenden Vertragsstaat : ES**

**1.** Verfahren zur Herstellung einer Verbindung der Formel I

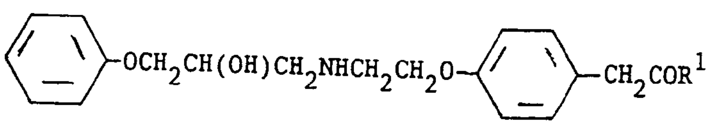

(I)

worin $R^1$ für Hydroxy oder $C_{1-10}$-Alkyl steht, oder eines in vivo hydrolysierbaren Esters oder pharmazeutisch zulässigen Salzes davon, bei welchem

a) eine Verbindung der Formel II oder der Formel III

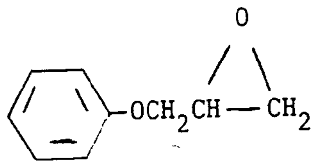

(II)

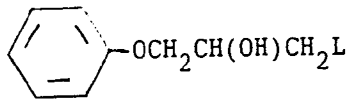

(III)

mit einer Verbindung der Formel IV

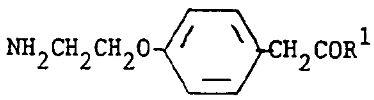

(IV)

umgesetzt wird, wobei $R^1$ die oben angegebene Bedeutung besitzt und L für eine Austrittsgruppe steht; oder

b) eine Verbindung der Formel V

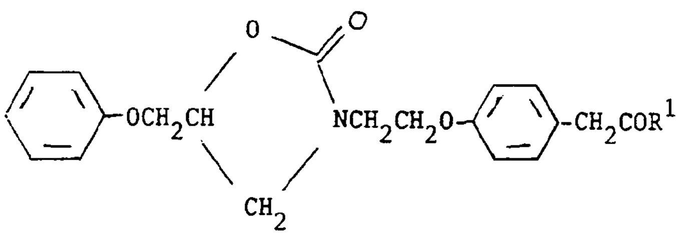

(V)

hydrolysiert wird, wobei $R^1$ die oben angegebene Bedeutung besitzt; oder

c) für Verbindungen der Formel I, worin $R^1$ für Hydroxy steht, eine Verbindung der Formel VI

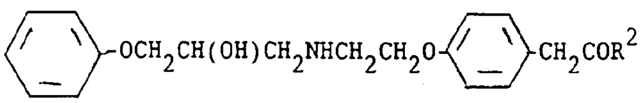

(VI)

hydrolysiert wird, wobei $R^2$ für eine hydrolysierbare Gruppe steht; oder

d) eine Verbindung der Formel VII

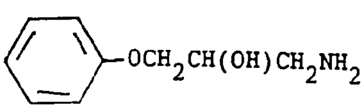

(VII)

mit einer Verbindung der Formel VIII

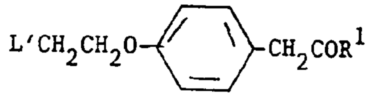

(VIII)

umgesetzt wird, wobei $R^1$ die oben angegebene Bedeutung besitzt und L' für eine Austrittsgruppe steht; oder

e) eine Verbindung der Formel IX

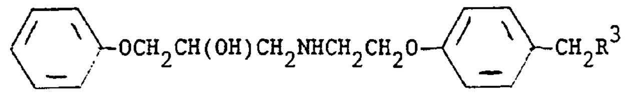

(IX)

worin $R^1$ die oben angegebene Bedeutung besitzt und $R^3$ für ein geschütztes Derivat von $-COR^1$ steht, von der Schutzgruppe befreit wird;

und hierauf gegebenenfalls ein in vivo hydrolysierbarer Ester oder ein pharmazeutisch zulässiges Salz hergestellt wird.

**2.** Verfahren zur Herstellung einer Verbindung der Formel I von Anspruch 1, worin $R^1$ für Hydroxy steht.

**3.** Verfahren zur Herstellung einer Verbindung der Formel I von Anspruch 1, worin $R^1$ für $C_{1-6}$-Alkyl steht.

**4.** Verfahren zur Herstellung einer Verbindung der Formel I von Anspruch 3, worin $R^1$ für Methyl, Ethyl, n-Propyl, Isobutyl, n-Butyl oder n-Hexyl steht.

**5.** Verfahren zur Herstellung einer Verbindung der Formel I von Anspruch 1, nämlich 4-[2-(2-Hydroxy-3-phenoxypropylarnino)ethoxy]phenylessigsäure und (S)-4-[2-(2-Hydroxy-3-phenoxypropylamino)ethoxy]phenylessigsäure.

**6.** Verfahren zur Herstellung einer Verbindung der Formel I von Anspruch 1, nämlich
1-[4-(2-(2-Hydroxy-3-phenoxypropylamino)ethoxy)phenyl]propan-2-on,
(S)-1-[4-(2-(2-Hydroxy-3-phenoxypropylamino)ethoxy)phenyl]propan-2-on,
(S)-1-[4-(2-(2-Hydroxy-3-phenoxypropylamino)ethoxy)phenyl]butan-2-on,
1-[4-(2-(2-Hydroxy-3-phenoxypropylamino)ethoxy)phenyl]pentan-2-on,
(S)-1-[4-(2-(2-Hydroxy-3-phenoxypropylamino)ethoxy)phenyl]pentan-2-on,
1-[4-(2-(2-Hydroxy-3-phenoxypropylamino)ethoxy)phenyl]-3-methylbutan-2-on,
1-[4-(2-(2-Hydroxy-3-phenoxypropylamino)ethoxy)phenyl]hexan-2-on,
1-[4-(2-(2-Hydroxy-3-phenoxypropylamino)ethoxy)phenyl]-4-methylpentan-2-on,
1-[4-(2-(2-Hydroxy-3-phenoxypropylamino)ethoxy)phenyl]heptan-2-on,
1-[4-(2-(2-Hydroxy-3-phenoxypropylamino)ehtoxy)phenyl]octan-2-on und
1-[4-(2-(2-Hydroxy-3-phenoxypropylamino)ethoxy)phenyl]nonan-2-on.

**7.** Verfahren zur Herstellung einer Verbindung der Formel I nach einem der Ansprüche 1 bis 6, welche an der Gruppe -CH(OH)-die absolute (S)-Konfiguration aufweist.

**8.** Verfahren zur Herstellung einer pharmazeutischen Zusammensetzung, bei welchem eine Verbindung der Formel I nach einem der Ansprüche 1 bis 7 und ein pharmazeutisch zulässiger Träger zusammengebracht werden.

**9.** Verbindungen der Formel VI

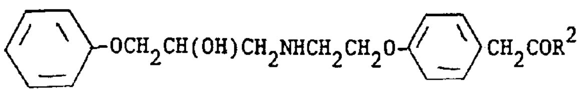

(VI)

worin $R^2$ für eine hydrolysierbare Gruppe steht.

10. Verbindungen nach Anspruch 9, worin $R^2$ für $C_{1-6}$-Alkoxy oder Amino ($-NH_2$) steht.

11. Verbindungen der Formel V

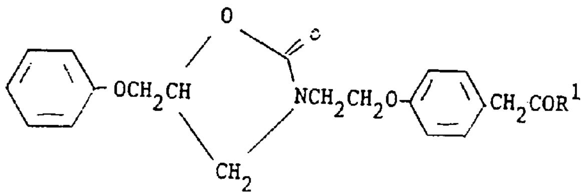

(V)

oder der Formel IX

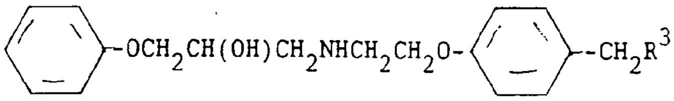

(IX)

worin $R^1$ die oben angegebene Bedeutung besitzt und $R^3$ für ein geschütztes Derivat von $-COR^1$ steht.

12. Verbindungen der Formel XIV

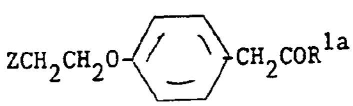

(XIV)

worin Z für Amino, geschütztes Amino oder eine Austrittsgruppe, wie z. B. Halogeno oder Sulfophenyloxy, steht und $R^{1a}$ für $C_{1-10}$-Alkyl steht, mit der Maßgabe, daß, wenn $R^{1a}$ für Methyl steht, Z nicht Amino oder geschütztes Amino bedeutet.

## Revendications

### Revendications pour les Etats contractants suivants : AT, BE, CH, DE, DK, FR, GB, IT, LI, LU, NL, SE

1. Composé de formule (I) :

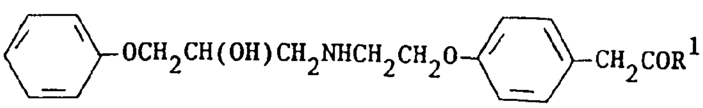

(I)

dans laquelle $R^1$ représente un groupe hydroxy ou alkyle en $C_{1-10}$, ou un de ses esters hydrolysables <u>in vivo</u> ou bien un de ses sels pharmaceutiquement acceptables.

2. Composé suivant la revendication 1, dans lequel $R^1$ représente un groupe hydroxy.

3. Composé suivant la revendication 1, dans lequel $R^1$ représente un groupe alkyle en $C_{1-6}$.

4. Composé suivant la revendication 3, dans lequel $R^1$ représente un groupe méthyle, éthyle, n-propyle, isobutyle, n-butyle ou n-hexyle.

5. Composé suivant la revendication 1, qui est : l'acide4-[2-(2-hydroxy-3-phénoxypropylamino)éthoxy]-phénylacétique ou l'acide (S)-4-[2-(2-hydroxy-3-phénoxypropylamino)éthoxy]-phénylacétique.

6. Composé suivant la revendication 1, qui est :
la 1-[4-(2-(2-hydroxy-3-phénoxypropylamino)éthoxy)phényl]propane-2-one,
la (S)-1-[4-(2-(2-hydroxy-3-phénoxypropylamino)éthoxy)phényl]propane-2-one,
la (S)-1-[4-(2-(2-hydroxy-3-phénoxypropylamino)éthoxy)phényl]butane-2-one,
la 1-[4-(2-(2-hydroxy-3-phénoxypropylamino)éthoxy)phényl]pentane-2-one,
la (S)-1-[4-(2-(2-hydroxy-3-phénoxypropylamino)éthoxy)phényl]pentane-2-one,
la 1-[4-(2-(2-hydroxy-3-phénoxypropylamino)éthoxy)phényl]3-méthylbutane-2-one,
la 1-[4-(2-(2-hydroxy-3-phénoxypropylamino)éthoxy)phényl]hexane-2-one,
la 1-[4-(2-(2-hydroxy-3-phénoxypropylamino)éthoxy)phényl]4-méthylpentane-2-one,
la 1-[4-(2-(2-hydroxy-3-phénoxypropylamino)éthoxy)phényl]heptane-2-one,
la 1-[4-(2-(2-hydroxy-3-phénoxypropylamino)éthoxy)phényl]octane-2-one ou,
la 1-[4-(2-(2-hydroxy-3-phénoxypropylamino)éthoxy)phényl]nonane-2-one.

7. Composé suivant l'une quelconque des revendications 1 à 6, lorsqu'il est sous la configuration absolue (S) au niveau du groupe -CH(OH)-.

8. Composition pharmaceutique qui comprend un composé suivant l'une quelconque des revendications 1 à 6 et un support pharmaceutiquement acceptable.

9. Composition pharmaceutique qui comprend un composé suivant la revendication 7 et un support pharmaceutiquement acceptable.

10. Procédé de préparation d'un composé de formule (I) ou d'un de ses esters hydrolysables in vivo ou sels pharmaceutiquement acceptables suivant la revendication 1, qui comprend :
a) la réaction d'un composé de formule (II) ou (III) avec un composé de formule (IV) :

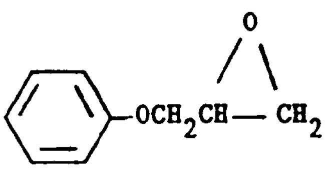

(II)

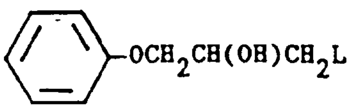

(III)

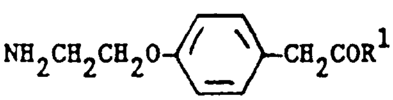

(IV)

formules dans lesquelles $R^1$ répond à la définition suivant la revendication 1 et L représente un groupe déplaçable ; ou
b) l'hydrolyse d'un composé de formule (V) :

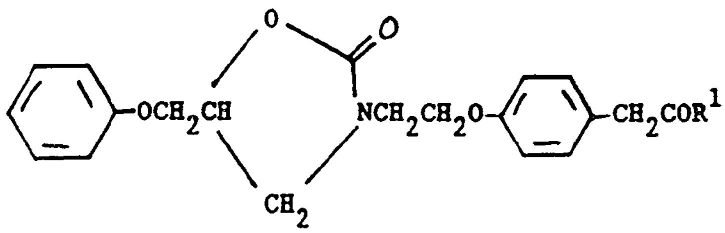

(V)

dans laquelle $R^1$ répond à la définition précitée ; ou

c) pour des composés de formule (I) dans laquelle $R^1$ représente un groupe hydroxy, l'hydrolyse d'un composé de formule (VI):

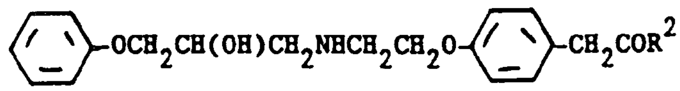

(VI)

dans laquelle $R^2$ représente un groupe hydrolysable ;

d) la réaction d'un composé de formule (VII) avec un composé de formule (VIII) :

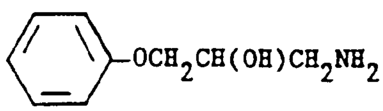

(VII)

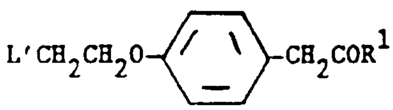

(VIII)

dans laquelle $R^1$ répond à la définition précitée et où L' représente un groupe partant ; ou

e) l'élimination de la protection d'un composé de formule (IX) :

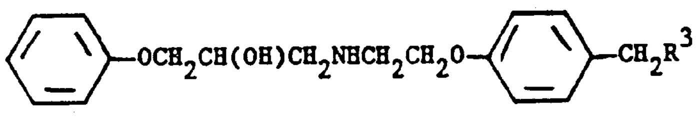

(IX)

dans laquelle $R^1$ répond à la définition précitée et $R^3$ représente un dérivé protégé du groupe $-COR^1$ ; et, si nécessaire, la formation ultérieure d'un ester hydrolysable in vivo ou d'un sel pharmaceutiquement acceptable.

**11.** Composé de formule (VI) :

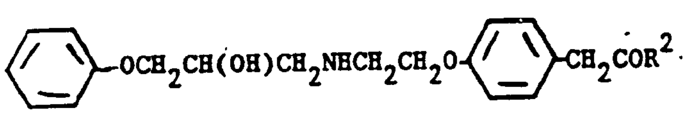

(VI)

dans laquelle $R^2$ représente un groupe hydrolysable .

**12.** Composé suivant la revendication 11, dans lequel $R^2$ représente un groupe alkoxy en $C_{1-6}$ ou amino ($NH_2$).

**13.** Composé de formule (V) :

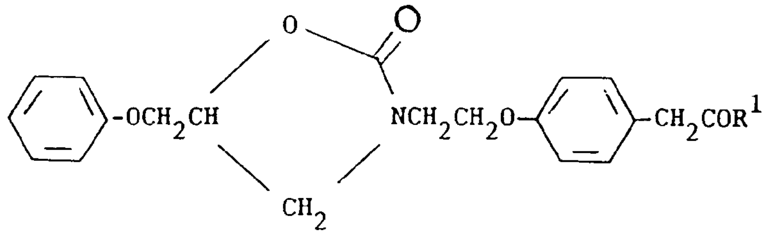

(V)

ou de formule (IX) :

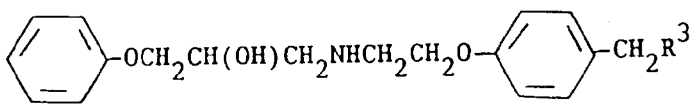

(IX)

formules dans lesquelles $R^1$ répond à la définition suivant la revendication 1 et $R^3$ représente un dérivé protégé du groupe $-COR^1$.

**14.** Composé de formule (XIV) :

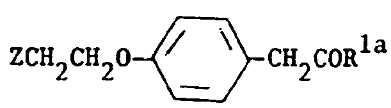

(XIV)

dans laquelle Z représente un groupe amino, un groupe amino protégé ou un groupe partant tel qu'un groupe halogéno ou sulfonyloxy et $R^{1a}$ représente un groupe alkyle en $C_{1-10}$, sous réserve que, lorsque $R^{1a}$ représente un groupe méthyle, Z ne soit pas un groupe amino ou amino protégé.

**Revendications pour l'Etat contractant suivant : ES**

**1.** Procédé de préparation d'un composé de formule (I) :

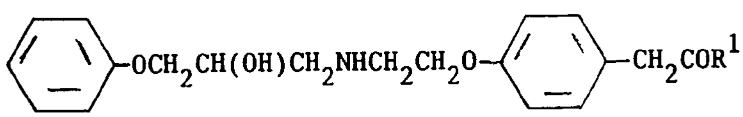

(I)

dans laquelle $R^1$ représente un groupe hydroxy ou alkyle en $C_{1-10}$, ou d'un de ses sels hydrolysables <u>in vivo</u> ou bien d'un de ses sels pharmaceutiquement acceptables procédé qui comprend :

a) la réaction d'un composé de formule (II) ou (III) avec un composé de formule (IV) :

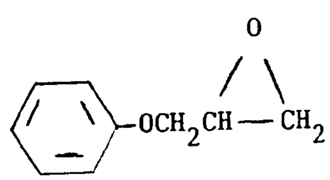

(II)

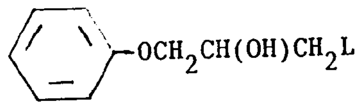

(III)

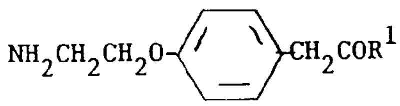

(IV)

formules dans lesquelles $R^1$ répond à la définition précitée et L représente un groupe déplaçable ; ou

b) l'hydrolyse d'un composé de formule (V) :

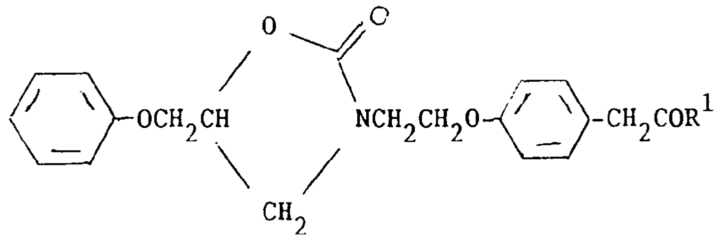

(V)

dans laquelle $R^1$ répond à la définition précitée ; ou

c) pour des composés de formule (I) dans laquelle $R^1$ représente un groupe hydroxy, l'hydrolyse d'un composé de formule (VI) :

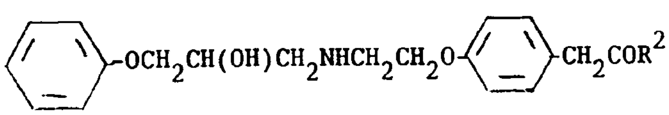

(VI)

dans laquelle $R^2$ représente un groupe hydrolysable ;

d) la réaction d'un composé de formule (VII) avec un composé de formule (VIII) :

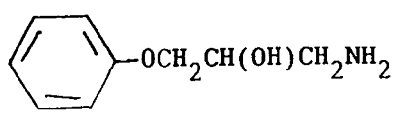

(VII)

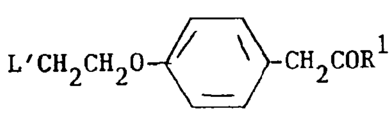

(VIII)

dans laquelle $R^1$ répond à la définition précitée et L' représente un groupe partant ; ou

e) l'élimination de la protection d'un composé de formule (IX) :

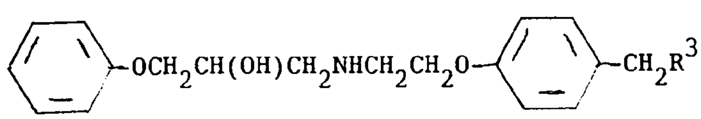

(IX)

dans laquelle $R^1$ répond à la définition précitée et $R^3$ représente un dérivé protégé du groupe $-COR^1$ ; et, si nécessaire, la formation ultérieure d'un ester hydrolysable <u>in vivo</u> ou d'un sel pharmaceutiquement acceptable.

2. Procédé de préparation d'un composé de formule (I) suivant la revendication 1, dans lequel $R^1$ représente un groupe hydroxy.

3. Procédé de préparation d'un composé de formule (I) suivant la revendication 1, dans lequel $R^1$ représente un groupe alkyle $C_{1-6}$.

4. Procédé de préparation d'un composé de formule (I) suivant la revendication 3, dans lequel $R^1$ représente un groupe méthyle, éthyle, n-propyle, isobutyle ou n-butyle, ou n-hexyle.

5. Procédé de préparation d'un composé de formule (I) suivant la revendication 1, qui est :
l'acide 4-[2-(2-hydroxy-3-phénoxypropylamino)éthoxy]-phénylacétique, ou
l'acide (S)-4-[2-(2-hydroxy-3-phénoxypropylamino)- éthoxy]-phénylacetique ;

6. Procédé de préparation d'un composé de formule (I) suivant la revendication 1, qui est
la 1-[4-(2-(2-hydroxy-3-phénoxypropylamino)éthoxy)phényl]propane-2-one,
la (S)-1-[4-(2-(hydroxy-3-phénoxypropylamino)éthoxy)phényl]propane-2-one,
la (S)-1-[4-(2-(2-hydroxy-3-phénoxypropylamino)éthoxy)phényl]butane-2-one,
la 1-[4-(2-(2-hydroxy-3-phénoxypropylamino)éthoxy)phényl]pentane-2-one,
la (S)-1-[4-(2-(2-hydroxy-3-phénoxypropylamino)éthoxy)phényl]pentane-2-one,
la 1-[4-(2-(2-hydroxy-3-phénoxypropylamino)éthoxy)phényl]-3-méthylbutane-2-one,
la 1-[4-(2-(2-hydroxy-3-phénoxypropylamino)éthoxy)phényl]hexane-2-one,
la 1-[4-(2-(2-hydroxy-3-phénoxypropylamino)éthoxy)phényl]-4-hylpentane-2-one,
la 1-[4-(2-(2-hydroxy-3-phénoxypropylamino)éthoxy)phényl]heptane-2-one,
la 1-[4-(2-(2-hydroxy-3-phénoxypropylamino)éthoxy)phényl]octane-2-one, ou
la 1-[4-(2-(2-hydroxy-3-phénoxypropylamino)éthoxy)phényl]nonane-2-one.

7. Procédé de préparation d'un composé de formule (I) suivant l'une quelconque des revendications 1 à 6, lorsqu'il est sous la configuration absolue (S) au niveau du groupe -CH(OH)-.

8. Procédé de préparation d'une composition pharmaceutique, qui comprend la mise en association d'un composé de formule (I) suivant l'une quelconque des revendications 1 à 7 et d'un support pharmaceutiquement acceptable.

9. Composé de formule (VI) :

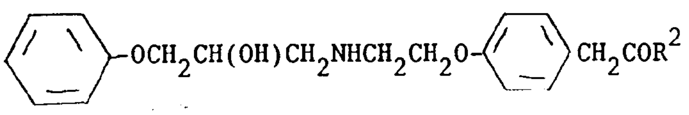

(VI)

dans laquelle $R^2$ représente un groupe hydrolysable.

10. Composé suivant la revendication 9, dans lequel $R^2$ représente un groupe alkoxy en $C_{1-6}$ ou amino ($-NH_2$).

11. Composé de formule (V) :

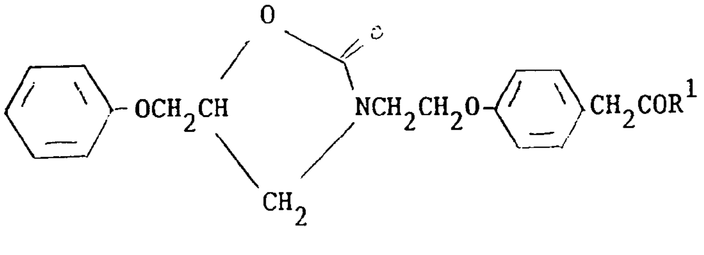

(V)

ou de formule (IX) :

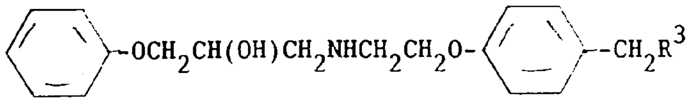

(IX)

formules dans lesquelles $R^1$ répond à la définition précitée et $R^3$ représente un dérivé protégé du groupe -$COR^1$.

**12.** Composé de formule (XIV) :

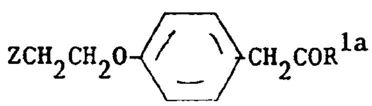

(XIV)

dans laquelle Z représente un groupe amino, un groupe amino protégé ou un groupe partant tel qu'un groupe halgéno ou sulfonyloxy et $R^{1a}$ représente un groupe alkyle en $C_{1-10}$ sous réserve que, lorsque $R^{1a}$ représente un groupe méthyle, Z ne soit pas un groupe amino ou amino protégé.